(19) Europäisches Patentamt
European Patent Office
Office européen des brevets

(11) EP 2 505 612 B1

(12) EUROPEAN PATENT SPECIFICATION

(45) Date of publication and mention of the grant of the patent:
**08.03.2017 Bulletin 2017/10**

(21) Application number: **12001779.3**

(22) Date of filing: **15.03.2012**

(51) Int Cl.:
***C08L 83/04*** *(2006.01)* ***A61K 8/89*** *(2006.01)*
***A61Q 19/00*** *(2006.01)* *A61Q 1/08 (2006.01)*
*A61Q 1/10 (2006.01)* *A61Q 15/00 (2006.01)*
*A61Q 17/04 (2006.01)* *C08G 77/12 (2006.01)*
*C08G 77/20 (2006.01)* *A61K 8/895 (2006.01)*
*A61K 8/02 (2006.01)*

# (54) ORGANOPOLYSILOXANE ELASTOMER COMPOSITION AND COSMETIC CONTAINING THE SAME

ORGANOPOLYSILOXANELASTOMERZUSAMMENSETZUNG UND KOSMETIKARTIKEL DAMIT

COMPOSITION D'ÉLASTOMÈRE ORGANOPOLYSILOXANE ET PRODUIT COSMÉTIQUE LA CONTENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.03.2011 JP 2011070451**

(43) Date of publication of application:
**03.10.2012 Bulletin 2012/40**

(73) Proprietor: **Shin-Etsu Chemical Co., Ltd.**
**Chiyoda-ku**
**Tokyo (JP)**

(72) Inventors:
- **Ikeda, Teruki**
**Annaka-shi**
**Gunma-ken (JP)**
- **Inokuchi, Yoshinori**
**Annaka-shi**
**Gunma-ken (JP)**
- **Horiguchi, Ryuji**
**Annaka-shi**
**Gunma-ken (JP)**
- **Inaba, Ryuichi**
**Tokyo (JP)**
- **Hayakawa, Chihiro**
**Tokyo (JP)**

(74) Representative: **Wibbelmann, Jobst**
**Wuesthoff & Wuesthoff**
**Patentanwälte PartG mbB**
**Schweigerstrasse 2**
**81541 München (DE)**

(56) References cited:
**EP-A2- 2 356 969**
**US-A- 5 538 793**
**US-A1- 2010 112 023**
**US-A1- 2010 112 074**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

BACKGROUND OF THE INVENTION

1. Field of the Invention

The present invention relates to a paste orgnopolysiloxane elastomer composition containing an organopolysiloxane elastomer microparticle, and to a cosmetic containing the same.

2. Description of the Related Art

An oil such as a silicone oil is used in various fields including cosmetics and foods because of its characteristics, safety, and so on. Especially in the fields of cosmetics for a skin care, a make-up, and so on, a silicone oil is blended thereinto with the aim to improve such effects as protective, softening, and moisturizing effects to a skin, and a use feeling. Especially a low-dynamic viscosity silicone oil having the dynamic viscosity of 100 or less $mm^2$/second is widely used, because of its excellent spreading properties and a refreshing feeling; but a silicone oil has been difficult to be blended into a cosmetic stably because of its poor compatibility with other cosmetic oil and low viscosity.

Accordingly, a proposal was made to blend a cosmetic with a paste composition obtained by mixing a specific silicone polymer with a low-viscosity silicone oil under a shear force (Japanese Patent No. 2582275). However, although the paste composition obtained by this method had an improved blending stability with a cosmetic, an oily feeling such as greasiness and oil-film feeling was felt on occasion. In U. S. Patent No. 5654362 and Japanese translation of PCT international application No. 2005-537364, an elastomer composition comprising a diluent and a crosslinked elastomer, which comprises a diene compound and a polysiloxane having an Si-H group is shown; but, the diene compound represented by 1,4-pentadiene and 1,5-hexadiene has an unpleasant unique odor, so that the composition thereof is not suitable in a cosmetic use if the diene compound is remained therein.

On the other hand, with the aim to improve use feelings such as smoothness and non-stickiness, a spherical silicone particle has been used. For example, a proposal was made to blend a cosmetic with a composition comprising a crosslinked spherical silicone particle having a specific particle diameter and a silicone oil (Japanese Patent No. 3488573 and Japanese Patent No. 4025454). In the methods shown therein, it could be seen the effects that use feelings such as smoothness and non-stickiness were improved, but a thickening effect of the spherical silicone particle to the silicone oil was insufficient, thereby requiring a large amount of the spherical silicone particle for blending and also causing separation of the silicone oil, agglomeration of the spherical silicone particles, and so on; and thus, in these methods there has been a problem of a storage stability and a blending stability with a cosmetic and so on.

SUMMARY OF THE INVENTION

The present invention was made in view of the situation as described above, and is intended to provide a paste organopolysiloxane elastomer composition having excellent stability in storage, blending, and so on with a cosmetic and so on while giving a sleek and light feeling without a heavy spreading property and without such feelings as an oily, a sticky, and an oil-film feeling; and to provide a cosmetic containing this composition.

To solve the problems mentioned above, the present invention provides, a paste organopolysiloxane elastomer composition comprising at least:

(I) an organopolysiloxane elastomer microparticle obtained by an addition polymerization of an organopolysiloxane mixture containing:

(i) an organohydrogen polysiloxane having at least two silicon-bonded hydrogen atoms in its molecular structure and shown by the following general formula (1),

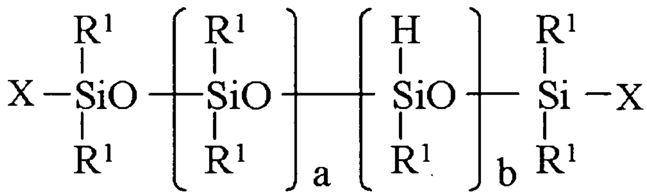

(1)

wherein each $R^1$ may be the same or different and represents a monovalent hydrocarbon group having 1 to 8 carbon atoms and not containing an aliphatic unsaturated group; "a" represents an integer of one or more, and "b" represents an integer of 0 or more, with a+b being an integer of 80 or more; and each X may be the same or different, and represents a hydrogen atom or $R^1$, and

(ii) an organopolysiloxane having at least two aliphatic unsaturated groups in its molecular structure and shown by the following general formula (2),

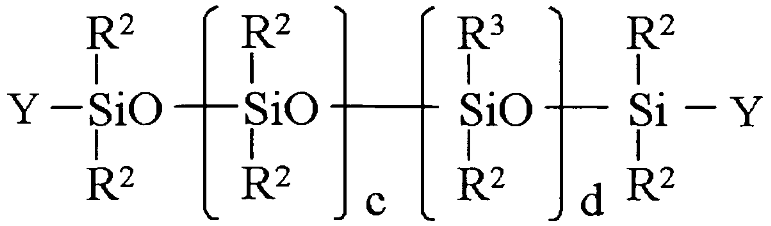
(2)

wherein each $R^2$ may be the same or different and represents a monovalent hydrocarbon group having 1 to 8 carbon atoms and not containing an aliphatic unsaturated group, and each $R^3$ may be the same or different and represents a monovalent hydrocarbon group having 2 to 8 carbon atoms and containing an aliphatic unsaturated group at its terminal; "c" represents an integer of one or more, and "d" represents an integer of 0 or more, with c+d being an integer of 80 or more; and each Y may be the same or different, and represents $R^2$ or $R^3$; and

(II) an oil that is a liquid at 25°C; wherein
the organopolysiloxane elastomer microparticle of the component (I) is a spherical or an amorphous microparticle having a volume-average particle diameter of 2 to 50 μm and can absorb 200 or more parts by mass of a methyl polysiloxane having a dynamic viscosity of 10 $mm^2$/second or less at 25°C relative to 100 parts by mass of the organopolysiloxane elastomer microparticle and the ratio of the frictional resistance force of the oil which is used for the organopolysiloxane elastomer composition to the frictional resistance force of the organopolysiloxane elastomer composition is 0.80 or more, and wherein the volume-average particle diameter is measured by a Coulter-counter method and the frictional resistance force is measured by a frictional resistance force-measuring apparatus which is capable of, at the time of frictioning an evaluation sample on a sample holder, measuring a frictional force of horizontal direction relative to the holder and a load of vertical direction relative to the holder at the same time.

The organopolysiloxane elastomer composition of the present invention as mentioned above has excellent stability in storage, blending, and so on with a cosmetic and so on while giving a sleek and light feeling without a heavy spreading property and without such feelings as an oily, a sticky, and an oil-film feeling.

In addition, the liquid oil of the component (II) is preferably at least one kind selected from an silicone oil, a hydrocarbon oil, an ester oil, a natural animal and vegetable oil, and a semi-synthetic oil.

The liquid oil as mentioned above is excellent in safety and so on; and thus, a composition obtained by blending the oil has a high safety, so that it may be used suitably in a cosmetic and so on.

Further, $R^3$ in the general formula (2) is preferably a vinyl group.

The organopolysiloxane elastomer microparticle obtained by an addition polymerization of an organopolysiloxane mixture containing an organopolysiloxane having an aliphatic unsaturated group, as mentioned above, is preferable because it shows a high thickening effect especially to the liquid oil.

In addition, the ratio of the frictional resistance force of the oil which is used for the organopolysiloxane elastomer composition to the frictional resistance force of the organopolysiloxane elastomer composition is 0.80 or more.

When the ratio of the frictional resistance force is 0.80 or more as described above, the paste organopolysiloxane composition has an excellent smoothness, lightness, spread-ability and the like.

In addition, the present invention provides a cosmetic, wherein the organopolysiloxane elastomer composition is contained therein.

The cosmetic like this has an excellent adhesion property and gives such feelings as a sleek, a powdery, and a refreshing feeling with a wide and a light spreading property and without such feelings as a greasy and a sticky feeling.

As mentioned above, the organopolysiloxane elastomer composition of the present invention gives a sleek feeling with a light spreading property and without an oily feeling and so on. Because of this, the cosmetic blended with this composition has characteristics of giving a sleek and a powdery feeling without an oily feeling.

DESCRIPTION OF THE PREFERRED EMBODIMENTS

Hereinafter, the present invention will be explained in more detail.

As mentioned above, a paste composition that has been previously used in a cosmetic and so on had various problems such as poor stability in storage, blending, and so on with a cosmetic and so on, a heavy spreading property, and such feelings as an oily, a sticky, and an oil-film feeling.

Accordingly, inventors of the present invention carried out an extensive investigation to solve the problems described above, and as a result, the inventors found that the problems described above could be solved by blending a liquid oil with an organopolysiloxane elastomer microparticle obtained by an addition polymerization of an organopolysiloxane mixture containing a specific organohydrogen polysiloxane and an organopolysiloxane having an aliphatic unsaturated group; and based on this finding, the present invention could be accomplished.

Namely, the present invention relates to a paste organopolysiloxane elastomer composition comprising at least:

(I) an organopolysiloxane elastomer microparticle obtained by an addition polymerization of an organopolysiloxane mixture containing:

(i) an organohydrogen polysiloxane having at least two silicon-bonded hydrogen atoms in its molecular structure and shown by the following general formula (1),

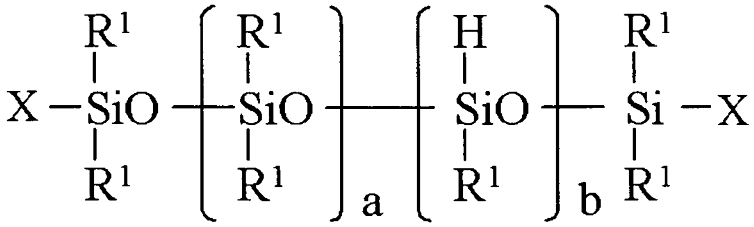

(1)

wherein each $R^1$ may be the same or different and represents a monovalent hydrocarbon group having 1 to 8 carbon atoms and not containing an aliphatic unsaturated group; "a" represents an integer of one or more, and "b" represents an integer of 0 or more, with a+b being an integer of 80 or more; and each X may be the same or different, and represents a hydrogen atom or $R^1$, and

(ii) an organopolysiloxane having at least two aliphatic unsaturated groups in its molecular structure and shown by the following general formula (2),

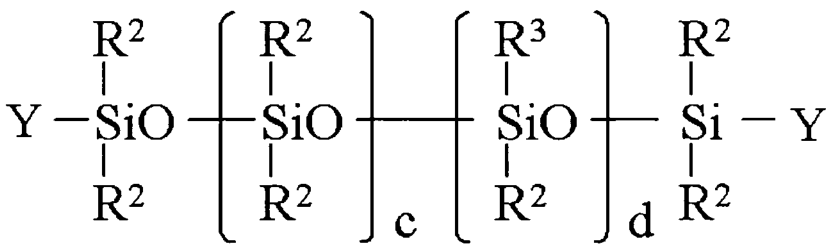

(2)

wherein each $R^2$ may be the same or different and represents a monovalent hydrocarbon group having 1 to 8 carbon atoms and not containing an aliphatic unsaturated group, and each $R^3$ may be the same or different and represents a monovalent hydrocarbon group having 2 to 8 carbon atoms and containing an aliphatic unsaturated group at its terminal; "c" represents an integer of one or more, and "d" represents an integer of 0 or more, with c+d being an integer of 80 or more; and each Y may be the same or different, and represents $R^2$ or $R^3$; and

(II) an oil that is a liquid at 25°C; wherein

the organopolysiloxane elastomer microparticle of the component (I) is a spherical or an amorphous microparticle having a volume-average particle diameter of 2 to 50 $\mu$m and can absorb 200 or more parts by mass of a methyl polysiloxane having a dynamic viscosity of 10 $mm^2$/second or less at 25°C relative to 100 parts by mass of the organopolysiloxane elastomer microparticle.

[(i) Organohydrogen polysiloxane]

In the general formula (1), each $R^1$ may be the same or different and represents a monovalent hydrocarbon group having 1 to 8 carbon atoms and not containing an aliphatic unsaturated group. Illustrative examples of $R^1$ include an alkyl group such as a methyl group, an ethyl group, a propyl group, a butyl group, a pentyl group, a hexyl group, a heptyl group, and an octyl group, as well as a phenyl group, wherein preferably 70% by mole or more relative to the total monovalent hydrocarbon groups represented by $R^1$ is a methyl group. The "a" represents an integer of one or more; but in view of a feeling and absorption of a methyl polysiloxane, "a" is preferably 80 to 900, or more preferably 100 to 500. The "b" represents an integer of zero or more, wherein "b" may be zero in the case that both terminals are hydrogen atoms, while "b" may be an integer of two or more in the case that both terminals are not hydrogen atoms. The "b" is preferably 0 to 40, or more preferably 0 to 20. X may be the same or different and represents a hydrogen atom or $R^1$, though preferably a hydrogen atom.

The organohydrogen polysiloxane in (i) may be used singly or as a mixture of two or more kinds thereof.

However, in the case that a+b in the general formula (1) is less than 80, absorption of a methyl polysiloxane is low; and thus, a large amount of the organopolysiloxane elastomer microparticle needs to be blended in order to obtain a paste composition, whereby this tends to give a heavy feeling and to separate the oil easily during storage. Accordingly, in the present invention, a+b in the general formula (1) needs to be an integer of 80 or more.

In view of productivity and so on, a+b is preferably $100 \leq a+b \leq 900$, or more preferably $100 \leq a+b \leq 500$.

[(ii) Organopolysiloxane having an aliphatic unsaturated group]

In the general formula (2), each $R^2$ may be the same or different and represents a monovalent hydrocarbon group having 1 to 8 carbon atoms and not containing an aliphatic unsaturated group. Illustrative examples of $R^2$ include an alkyl group such as a methyl group, an ethyl group, a propyl group, a butyl group, a pentyl group, a hexyl group, a heptyl group, and an octyl group, as well as a phenyl group, wherein preferably 70% by mole or more of the total monovalent hydrocarbon groups represented by $R^2$ is a methyl group. Each $R^3$ may be the same or different and represents a monovalent hydrocarbon group having 2 to 8 carbon atoms and containing an aliphatic unsaturated group at its terminal. Illustrative examples of $R^3$ include a vinyl group, an allyl group, a propenyl group, a butenyl group, a pentenyl group, and a hexenyl group, while a vinyl group is preferable. The "c" represents an integer of one or more; but in view of a feeling and absorption of a methyl polysiloxane, "c" is preferably 80 to 900, or more preferably 100 to 500. The "d" represents an integer of zero or more, wherein "d" may be zero in the case that both terminals are $R^3$, while "d" may be an integer of two or more in the case that both terminals are not $R^3$. The "d" is preferably 0 to 40, or more preferably 0 to 20. For the same reason as a+b in the general formula (1), in the present invention, c+d in the above general formula needs to be an integer of 80 or more, preferably $100 \leq c+d \leq 900$, or more preferably $100 \leq c+d \leq 500$. Y represents $R^2$ or $R^3$, or preferably $R^3$.

The organopolysiloxane having an aliphatic unsaturated group in (ii) may be used singly or as a mixture of two or more kinds thereof.

[(I) Organopolysiloxane elastomer microparticle]

The organopolysiloxane elastomer microparticle in the component (I) is obtained by an addition polymerization of an organopolysiloxane mixture containing an organohydrogen polysiloxane shown by the general formula (1) and an organopolysiloxane having an aliphatic unsaturated group and shown by the general formula (2).

The organopolysiloxane elastomer microparticle of the present invention may be spherical or amorphous, but is preferably a spherical microparticle. The organopolysiloxane elastomer microparticle has the volume-average particle diameter of 2 to 50 μm, preferably 3 to 40 μm, or more preferably 5 to 35 μm, wherein the diameter is measured before swelling by the oil. When the volume-average particle diameter thereof is smaller than 2 μm, the spreading property thereof tends to be heavy, while the average-volume particle diameter is larger than 50 μm, a rough and an oil-film feeling tend to appear.

Meanwhile, the volume-average particle diameter is measured by a Coulter-counter method. The term "spherical" means that the organopolysiloxane elastomer microparticle is not only in the form of a perfect sphere, but also in the form of a deformed sphere having a ratio of a length of the longest axis to a length of the shortest axis (aspect ratio) of 1 to 4, preferably 1 to 2, more preferably 1 to 1.6, or still more preferably 1 to 1.4, as an average. This form of the microparticle can be confirmed by observation with an optical microscope or an electron microscope.

The rubber hardness of the organopolysiloxane elastomer microparticle, measured with a Type A durometer according to JIS K 6253, is preferably in the range of 5 to 90, or more preferably in the range of 10 to 80, in view of not only fluidity and dispersibility but also a feeling such as sleekness and smoothness.

The organopolysiloxane elastomer microparticle is the one that can absorb 200 parts by mass or more (oil-absorption) of a methyl polysiloxane having the dynamic viscosity of 10 $mm^2$/second or less at 25°C, based on 100 parts by mass of the organopolysiloxane elastomer particle. When the oil-absorption amount is less than 200 parts by mass, it is difficult to eliminate a sticky feeling and an oil-film feeling from a cosmetic blended with the microparticle.

Meanwhile, the methyl polysiloxane having the dynamic viscosity of 10 $mm^2$/second or less may be in any of a linear, a cyclic, and a branched structure; illustrative examples of the methyl polysiloxane include a dimethyl polysiloxane shown by the formula $(CH_3)_3SiO[(CH_3)_2SiO]_nSi(CH_3)_3$ (n=1 to 15); a cyclic siloxane such as octamethyl cyclotetrasiloxane, decamethyl cyclopentasiloxane, and dodecamethyl cyclohexasiloxane; and a branched siloxane such as tristrimethyl siloxymethyl silane and tetrakistrimethylsiloxy silane.

The oil-absorption amount may be obtained, for example, as following: an organopolysiloxane elastomer microparticle and a methyl polysiloxane, the mass of them being measured in advance, are taken into a glass bottle or the like, shaken for several tens of minutes, and allowed to stand for several days at room temperature; and then, after the solid portion is separated therefrom by a filter press, the mass of the organopolysiloxane elastomer microparticle is subtracted from the mass of the solid portion.

The organopolysiloxane elastomer microparticle (I) of the present invention shows a high thickening effect to the liquid oil such as a silicone oil and also has an excellent storage stability and so on.

[Preparation method of the organopolysiloxane elastomer microparticle]

The organopolysiloxane elastomer microparticle can be prepared, as mentioned above, by an addition polymerization of the organohydrogen polysiloxane shown by the general formula (1) and the organopolysiloxane having an aliphatic unsaturated group and shown by the general formula (2); and the addition polymerization is carried out at a temperature of 20 to 150°C in the presence of a catalyst known to those skilled in the art for an addition reaction, for example, platinum compounds such as chloroplatinic acid, an alcohol-modified chloroplatinic acid, and a chloroplatinic acid-vinyl siloxane complex; and metal compounds belonging to the platinum group including rhodium palladium, and the like. The catalyst for the addition reaction may be used with an effective amount known to those skilled in the art; and thus, the amount is usually 0.1 to 500 ppm, preferably 0.5 to 200 ppm, or more preferably 1 to 100 ppm, in terms of the converted mass to the platinum group metal, relative to the totality of the composition.

Alternatively, the organopolysiloxane elastomer microparticle may also be obtained as following: into a mixture of the organohydrogen polysiloxane shown by the general formula (1) and the organopolysiloxane having an aliphatic unsaturated group and shown by the general formula (2) were added a surfactant and water, and then, after the resulting mixture is emulsified, the addition reaction is carried out by adding the afore-mentioned catalyst for the addition reaction into the emulsified mixture to produce an aqueous dispersion solution of the organopolysiloxane elastomer particle; and then, water is removed from the emulsion thereby obtained.

As to the surfactant, there are an anionic, a cationic, a nonionic, and an amphoteric surfactant; and in the present invention, there is no particular restriction, and thus, any of them may be used provided that the surfactant is used in a usual cosmetic.

Illustrative examples of the anionic surfactant include an alkyl sulfate, a polyoxyethylene alkyl ether sulfate, a polyoxyethylene alkyl phenyl ether sulfate, an N-acyl taurate, an alkylbenzene sulfonate, a polyoxyethylene alkyl phenyl ether sulfonate, an $\alpha$-olefin sulfonate, an alkylnaphthalene sulfonate, an alkyl diphenyl ether disulfonate, a dialkyl sulfosuccinate, a monoalkyl sulfosuccinate, a polyoxyethylene alkyl ether sulfosuccinate, an fatty acid salt, a polyoxyethylene alkyl ether acetate, an N-acyl amino acid salt, an alkenylsuccinate, an alkylphosphate, a polyoxyethylene alkyl ether phosphate, a polystyrene sulfonate, a condensation product of a naphthalene sulfonic acid with formalin, a condensation product of an aromatic sulfonic acid with formalin, a carboxylic acid polymer, and a copolymer of styrene with an oxyalkylene acid anhydride.

Illustrative examples of the cationic surfactant include an alkyl trimethyl ammonium salt, a dialkyl dimethyl ammonium salt, a polyoxyethylene alkyl dimethyl ammonium salt, a dipolyoxyethylene alkyl methyl ammonium salt, a tripolyoxyethylene alkyl ammonium salt, an alkyl benzyl dimethyl ammonium salt, an alkyl pyridinium salt, a monoalkyl amine salt, a monoalkylamide amine salt, and a cationic cellulose.

Illustrative examples of the nonionic surfactant include a polyoxyethylene alkyl ether, a polyoxyethylene polyoxypropylene alkyl ether, a polyoxyethylene alkylphenyl ether, a polyethylene glycol fatty acid ester, a sorbitan fatty acid ester, a polyoxyethylene sorbitan fatty acid ester, a polyoxyethylene sorbit fatty acid ester, a glycerin fatty acid ester, a polyoxyethylene glycerin fatty acid ester, a polyglycerin fatty acid ester, a propylene glycol fatty acid ester, a polyoxyethylene castor oil, a polyoxyethylene hard castor oil, a fatty acid ester of a polyoxyethylene hard castor oil, a polyoxyethylene alkyl amine, and a polyoxyethylene fatty acid amide.

Illustrative examples of the amphoteric surfactant include a betaine, an aminocarboxylic acid salt, an imidazoline derivative, and an amide amine type.

The emulsification may be done by using a heretofore known emulsification disperser such as a propeller mixer, a puddle mixer, a homomixer, a homodisper, a sand grinder, a colloid mill, a homogenizer, and an ultramixer.

In the case that dispersion of the catalyst for the addition reaction into water is poor, it is preferable that the catalyst be added into the emulsion in the state that the catalyst is dissolved into the foregoing surfactant. The addition reaction may be carried out at room temperature; but if the reaction is not completed, heating may be done below 100°C.

Removal of water from the aqueous dispersion solution of the organopolysiloxane elastomer microparticle may be done, for example, by heating the aqueous dispersion solution under a normal pressure or a reduced pressure; specifically, a method to remove water by heating the aqueous dispersion solution under a static condition, a method to remove water by heating with stirring to fluidize the aqueous dispersion solution, a method to remove water by spraying the dispersion solution into a heated air stream such as a spray dryer, and so on, may be used. Meanwhile, as a pretreatment of the water removal by these methods, the aqueous dispersion solution may be concentrated by such methods as dehydration by heating, separation by filtration, centrifugal separation, and decantation. In addition, rinsing by water may be done as necessary.

If the organopolysiloxane elastomer microparticle obtained by removing water from the aqueous dispersion solution is clumped together, the clump may be crushed by a crusher such as a jet mill, a ball mill, and a hammer mill.

[(II) Liquid oil]

As to the liquid oil used as the component (II) of the present invention, the one having the dynamic viscosity of 0.65 to 10000 $mm^2$/second measured at 25°C is preferable. The liquid oil like this may be at least one kind selected from a silicone oil, a hydrocarbon oil, an ester oil, a natural animal and vegetable oil, a semi-synthetic oil, and so on.

Illustrative examples of the silicone oil include an organopolysiloxane, having the dynamic viscosity of 0.65 to 10000 $mm^2$/second or preferably 0.65 to 1000 $mm^2$/second, such as dimethyl polysiloxane, methyl phenyl polysiloxane, and dimethylsiloxane/methyl phenyl siloxane copolymer; a cyclic siloxane such as octamethyl cyclotetrasiloxane, decamethyl cyclopentasiloxane, and dodecamethyl cyclohexasiloxane; a branched siloxane such as tristrimethyl siloxymethyl silane and tetrakistrimethyl siloxysilane; caprylyl methicone, an alkyl-modified silicone, an amino-modified silicone, and a fluorine-modified silicone.

Illustrative examples of the hydrocarbon oil include a chain or a cyclic hydrocarbon such as an $\alpha$-olefin oligomer, a light isoparaffin, a light liquid isoparaffin, squalane, a synthetic squalane, a vegetable squalane, a liquid paraffin, a liquid isoparaffin, a hydrogenated isobutene, isododecane, and isohexadecane.

Illustrative examples of the ester oil include dioctyl succinate, diisobutyl adipate, dioctyl adipate, di(2-heptylundecyl) adipate, diisopropyl sebacate, dioctyl sebacate, dibutyloctyl sebacate, diisostearyl malate, triethyl citrate, ethylene glycol dioctanoate, neopentyl glycol dioctanoate, propylene glycol dicaprate, neopentyl glycol dicaprate, trimethylolpropane trioctanoate, trimethylolpropane triisostearate, pentaerythritol tetraoleate, ethyl acetate, butyl acetate, amyl acetate, octyldodecyl neopentanoate, cetyl octanoate, isononyl isononanoate, isotridecyl isononanoate, hexyldecyl dimethyloctanoate, ethyl laurate, hexyl laurate, isopropyl myristate, myristyl myristate, isocetyl myristate, octyldodecyl myristate, isopropyl palmitate, octyl palmitate, cetyl palmitate, isocetyl palmitate, isostearyl palmitate, butyl stearate, hexyldecyl stearate, isopropyl isostearate, isocetyl isostearate, decyl oleate, oleil oleate, octyldodecyl oleate, ethyl linoate, isopropyl linoate, cetyl lactate, myristyl lactate, cholesteryl hydroxystearate, dioctyldodecyl lauroylglutamate, and isopropyl lauroylsarcosinate.

Illustrative examples of the natural animal and vegetable oil and the semi-synthetic oil include an avocado oil, an almond oil, an olive oil, a wheat germ oil, a sesame oil, a rice germ oil, a rice bran oil, a sugarcane wax, a sasanqua oil, a safflower oil, a cinnamon oil, squalane, squalene, a turtle oil, a soybean oil, a tea seed oil, a camellia oil, an evening primrose oil, a corn oil, a rapeseed oil, a Japanese tung oil, a germ oil, a sunflower oil, a grape seed oil, a jojoba oil, a macademia nut oil, a cotton seed oil, a coconut oil, a hardened coconut oil, a tri-coconut fatty acid glyceride, a peanut oil, and an egg-yolk oil.

[Paste organopolysiloxane elastomer composition]

To obtain the paste organopolysiloxane elastomer composition (hereinafter, sometimes called merely "paste composition") from the organopolysiloxane elastomer microparticle (I) of the present invention, it is preferable that, after mixing the microparticle with the liquid oil at 25°C (room temperature), kneading of the resulting mixture be carried out under a shear force, so that the paste composition having a smooth appearance may be obtained. Kneading under a shear force may be carried out by using, for example, a three-roll mill, a two-roll mill, a sand grinder, a colloid mill, a homogenizer, an ultramixer, a homomixer, and a homodisper, while a three-roll mill and a homodisper are preferable.

The mixing ratio of the organopolysiloxane microparticle (I) and the liquid oil (II) in the present invention is preferably 1/20 to 20/1 (mass ratio), or more preferably 1/10 to 1/1.

<Slipperiness>

As for the organopolysiloxane composition, the ratio of the frictional resistance force of the oil which is used for the organopolysiloxane elastomer composition to the frictional resistance force of the organopolysiloxane elastomer composition ("slipperiness") is 0.80 or more.

This "slipperiness" is measured by a frictional resistance force-measuring apparatus which is capable of, at the time of frictioning an evaluation sample on a sample holder, measuring a frictional force of horizontal direction relative to the holder and a load of vertical direction relative to the holder at the same time.

As the frictional resistance force-measuring apparatus which is capable of, at the time of frictioning the evaluation sample on the sample holder, measuring a frictional force of horizontal direction relative to the holder and a load of vertical direction relative to the holder at the same time, "Portable tactile meter TYPE:33 (manufactured by SHINTO scientific Co. Ltd.)" can be exemplified. The measurement is preferably carried out by setting an artificial leather on the sample holder. As the artificial leather to be used, "Artificial Leather SUPPLALE (made by IDEMITSU TECHNOFINE Co. Ltd.)", which has human skin-like feel and asperity, is preferably used. The measurement may be carried out by frictioning with a finger(s) or by using a jig which keeps touch area constant, in the latter case, an artificial leather is preferably set to a touch surface of the jig.

Test sample is applied onto the artificial leather on the sample holder, and then, the frictional resistance force is measured when the reciprocatory actions are conducted along horizontal direction with a finger(s) or the jig. The closer the frictional force of the paste organopolysiloxane elastomer composition is to the frictional resistance force of the only liquid oil which constitutes the paste organopolysiloxane elastomer composition, the more sufficiently the composition keeps paste-form while keeping the feel of the oil. The smaller frictional force the organopolysiloxane elastomer composition has, the more excellent smoothness, lightness, spread ability and the like the composition shows.

From this, by defining "the frictional resistance force of the oil which is used for the organopolysiloxane elastomer composition /the frictional resistance force of the organopolysiloxane elastomer composition" as "slipperiness", it becomes possible to evaluate the smoothness, lightness, spread-ability and the like of the paste organopolysiloxane elastomer composition quantitatively. In the case of "slipperiness" is 0.8 or more, there is no fear of poor lightness and too low spread-ability, therefore it is preferable that "slipperiness" $\geq$ 0.8, more preferable that "slipperiness" $\geq$ 0.9.

[Cosmetic]

In addition, the present invention relates to a cosmetic containing therein the paste composition of the present invention. This cosmetic may contain, for example, in addition to the paste composition of the present invention, at least one kind, allowed as a cosmetic component, namely, selected from the group consisting of (A) an oil, (B) water, (C) a compound having an alcoholic hydroxyl group, (D) a water-soluble or a water-swelling polymer, (E) a heretofore known powder other than the organopolysiloxane elastomer microparticle of the present invention, (F) a surfactant, (G) a conventional silicone resin, and (H) a paste composition other than the paste composition of the present invention; the paste composition here comprising a crosslinking organopolysiloxane polymer and a liquid oil that are in the public domain.

The oil of the component (A) may be in the form of any of a solid, a semi-solid, and a liquid. As to the liquid oil, the same liquid oil as those used in the organopolysiloxane elastomer composition of the present invention mentioned above, other liquid oil, or a mixture of them may be used. Specific examples of the liquid oil are those as described before.

Illustrative examples of the other oil include a linseed oil, an insects wax, a perilla oil, a cocoa butter, a kapok wax, a kaya oil, a carnauba wax, a lever oil, a candellila wax, a purified candellila wax, a beef tallow, a neats-foot oil, a beef bone fat, a hardened beef tallow, an apricot kernel oil, a whale wax, a hydrogenated oil, a shea butter, a Chinese tung oil, a jojoba wax, a shellac wax, a lard, a horse fat, a bran wax, a persic oil, a palm oil, a palm kernel oil, a castor oil, a hardened castor oil, a methyl ester of castor oil fatty acid, a bayberry wax, a bees wax, a mink oil, a meadowfoam oil, a mutton tallow, a cotton wax, a Japan wax, a Japan wax kernel oil, a montan wax, lanolin, liquid lanolin, reduced lanolin, lanolin alcohol, hard lanolin, lanolin acetate, lanolin alcohol acetate, isopropyl lanolin fatty acid, polyoxyethylene lanolin alcohol ether, polyoxyethylene lanolin alcohol acetate, polyethylene glycol lanolin fatty acid, and polyoxyethylene hydrogenated lanolin alcohol ether.

In addition, included therein are a hydrocarbon oil, a higher fatty acid, a higher alcohol, a fluorinated oil, and so on. Illustrative examples of the hydrocarbon oil include an ozokerite, a ceresin, a paraffin, a paraffin wax, a polyethylene wax, a polyethylene/polypropylene wax, a pristane, polyisobutylene, a microcrystalline wax, and vaseline. Illustrative examples of the higher fatty acid include lauric acid, myristic acid, palmitic acid, stearic acid, behenic acid, isostearic acid, and 12-hydroxystearic acid. Illustrative examples of the higher alcohol include an alcohol having preferably 6 or more, or more preferably 10 to 30 carbon atoms. Specific examples of the higher alcohol include lauryl alcohol, myristyl alcohol, palmityl alcohol, stearyl alcohol, behenyl alcohol, hexadecyl alcohol, oleyl alcohol, isostearyl alcohol, hexyl dodecanol, octyl dodecanol, cetostearyl alcohol, 2-decyl tetradecynol, cholesterol, phytosterol, polyoxyethylene cholesterol ether, monostearyl glycerine ether (batyl alcohol), and monooleyl glyceryl ether (selachyl alcohol). Illustrative examples of the fluorinated oil include perfluoro polyether, perfluoro decalin, and perfluoro octane.

Amount of the oil of the component (A) to be blended is chosen appropriately dependent on the form of the cosmetic of the present invention; but it is preferable in the range of 1 to 98% by mass relative to the totality of the cosmetic.

Amount of water of the component (B) to be blended is chosen appropriately dependent on the form of the cosmetic of the present invention; but it is preferable in the range of 1 to 95% by mass relative to the totality of the cosmetic.

Illustrative examples of the compound having an alcoholic hydroxyl group of the component (C) include a lower alcohol preferably having 2 to 5 carbon atoms (a lower monovalent alcohol) such as ethanol and isopropanol, and a sugar alcohol such as sorbitol and maltose. In addition, included therein are a sterol such as cholesterol, sitosterol, phytosterol, and lanosterol; and a polyvalent alcohol such as butylene glycol, propylene glycol, dibutylene glycol, and pentylene glycol.

Amount of the component (C) to be blended is chosen preferably and appropriately in the range of 0.1 to 98% by mass relative to the totality of the cosmetic.

Illustrative examples of the water-soluble or the water-swelling polymer of the component (D) include a vegetable polymer such as an gum arabic, tragacanth, galactan, a carob gum, a guar gum, a karaya gum, carrageenan, pectin, agar, quince seed (marmelo), starch (rice, corn, potato, wheat, and so on), algae colloid, a tranto gum, and a locust bean gum; a microbial polymer such as a xanthan gum, dextran, succinoglucan, and pullulan; an animal polymer such as collagen, casein, albumin, and gelatin; a starch polymer such as carboxymethyl starch and methylhydroxypropyl starch; a cellulose polymer such as methyl cellulose, ethyl cellulose, methyl hydroxypropyl cellulose, carboxymethyl cellulose, hydroxymethyl cellulose, hydroxypropyl cellulose, nitrocellulose, sodium cellulose sulfate, sodium carboxymethyl cellulose, crystalline cellulose, and powdery cellulose; an alginic acid polymer such as sodium alginate and propylene glycol alginate ester; a vinyl polymer such as polyvinyl methyl ether and carboxy vinyl polymer; a polyoxyethylene polymer; a polyoxyethylene polyoxypropylene copolymer; an acryl polymer such as sodium polyacrylate, polyethyl acrylate, polyacrylamide, and acryloyldimethyl taurate salt copolymer; other water-soluble polymer such as polyethyleneimine and an cationic polymer; and an inorganic water-soluble polymer such as bentonite, aluminum magnesium silicate, montmorillonite, beidellite, nontronite, saponite, hectorite, and silicic acid anhydride. In addition, included therein are a film-forming material such as polyvinyl alcohol and polyvinyl pyrrolidone.

Amount of the component (D) to be blended is preferably in the range of 0.1 to 25% by mass relative to the totality of the cosmetic of the present invention.

As to the heretofore known powder of the component (E) other than the organopolysiloxane elastomer microparticle of the present invention, any powder may be used regardless of its form (spherical, needle-like, plate-like, and so on), its particle diameter (fumed, microparticle, pigment-class, and so on), and its particle structure (porous, non-porous, and so on), provided that the powder is approved to be used in a usual cosmetic. Illustrative examples of the powder include an inorganic powder, an organic powder, a surfactant metal salt powder, a colored pigment, a pearl pigment, a metal powder pigment, a tar dye, and a natural dye.

Illustrative examples of the inorganic powder include a microparticle of titanium oxide, titanium mica, zirconium oxide, zinc oxide, cerium oxide, magnesium oxide, barium sulfate, calcium sulfate, magnesium sulfate, calcium carbonate, magnesium carbonate, talc, cleaved talc, mica, kaolin, sericite, white mica, synthetic mica, golden mica, red mica, black mica, lithia mica, silicic acid, silicon dioxide, fumed silica, hydrated silicon dioxide, aluminum silicate, magnesium silicate, aluminum magnesium silicate, calcium silicate, barium silicate, strontium silicate, a metal salt of tungstic acid salt, hydroxy apatite, vermiculite, haidilite, bentonite, montomorillonite, hectorite, zeolite, ceramics, dibasic calcium phosphate, alumina, aluminum hydroxide, boron nitride, and glass.

Illustrative examples of the organic powder include a polyamide, polyacrylic acid, a polyacrylate ester, a polyester, polyethylene, polypropylene, polystyrene, styrene-acrylic acid copolymer, divinyl benzene-styrene copolymer, a polyurethane, a vinyl resin, an urea resin, a melamine resin, bezoguanamine, polymethyl benzoguanamine, tetrafluoroethylene, a polymethylmethacrylate (such as polymethacrylate methyl), cellulose, silk, nylon, a phenolic resin, an epoxy resin, polycarbonate, a silicone elastomer particle, polymethyl silsesquioxane particle, and a powder obtained by coating surface of the silicone elastomer particle with polymethyl silsesquioxane described in Japanese Patent No. 2832143.

Illustrative examples of the surfactant metal salt powder include a powder of zinc stearate, aluminum stearate, calcium stearate, magnesium stearate, zinc myristate, magnesium myristate, zinc cetylphosphate, calcium cetylphosphate, and zinc sodium cetylphosphate.

Illustrative examples of the colored pigment include an inorganic red pigment such as iron oxide, iron hydroxide, and iron titanate; an inorganic brown pigment such as $\gamma$-iron oxide; an inorganic yellow pigment such as a yellow iron oxide and a loess; an inorganic black pigment such as a black iron oxide and a carbon black; an inorganic purple pigment such as a manganese violet and a cobalt violet; an inorganic green pigment such as chromium hydroxide, chromium oxide, cobalt oxide, and cobalt titanate; an inorganic blue pigment such as Prussian blue and ultramarine blue; a laked tar dye; and a laked natural dye.

Specific examples of the pearl pigment include a mica covered with titanium oxide, bismuth oxychloride, oxychloro bismuth covered with titanium oxide, a talc covered with titanium oxide, a fish scale foil, and a colored mica

covered with titanium oxide.

Illustrative examples of the metal powder pigment include an aluminum powder, a copper powder, and a stainless powder. Illustrative examples of the tar dye include Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, and Orange No. 207. Illustrative examples of the natural dye include carminic acid, laccaic acid, carthamin, brazilin, and crocin.

Among these powders, preferable in the present invention are a spherical crosslinking dimethyl polysiloxane microparticle having a structure of at least a part of the dimethyl polysiloxane being crosslinked, a crosslinking polymethyl silsesquioxane microparticle, and a microparticle obtained by coating surface of a spherical crosslinking polysiloxane rubber with polymethyl silsesquioxane particle; and in addition, any one of a powder having a fluorine group and a coloring agent or both are used widely. In addition, usable as these powders are powder composites; a powder treated with a general oil, a silicone oil, a fluorine-containing compound, a surfactant, and the like; a reactive organohydrogen polysiloxane; an organopolysiloxane having a hydrolyzable alkoxysilane group; an acryl-silicone copolymer having a hydrolysable silyl group; and so on; or, as appropriate, one, or two or more of them.

Amount of these powders to be blended is preferably 0.1 to 99% by mass relative to the totality of the cosmetic. Especially in the case of a powdery solid cosmetic, the amount thereof is preferably 80 to 99% by mass relative to the totality of the cosmetic.

As to the surfactant of the component (F), there are an anionic, a cationic, a nonionic, and an amphoteric surfactant; and in the present invention, there is no particular restriction, and thus any of them may be used provided that the surfactant is used in a usual cosmetic.

Illustrative examples of the anionic surfactant include an alkyl sulfate, a polyoxyethylene alkyl ether sulfate, a polyoxyethylene alkyl phenyl ether sulfate, an N-acyl taurate, an alkylbenzene sulfonate, a polyoxyethylene alkyl phenyl ether sulfonate, an $\alpha$-olefin sulfonate, an alkylnaphthalene sulfonate, an alkyl diphenyl ether disulfonate, a dialkyl sulfosuccinate, a monoalkyl sulfosuccinate, a polyoxyethylene alkyl ether sulfosuccinate, a fatty acid salt, a polyoxyethylene alkyl ether acetate, an N-acyl amino acid salt, an alkenylsuccinate, an alkylphosphate, a polyoxyethylene alkyl ether phosphate, a polystyrene sulfonate, a condensation product of a naphthalene sulfonic acid with formalin, a condensation product of an aromatic sulfonic acid with formalin, a carboxylic acid polymer, and a copolymer of styrene with an oxyalkylene acid anhydride.

Illustrative examples of the cationic surfactant include an alkyl trimethyl ammonium salt, a dialkyl dimethyl ammonium salt, a polyoxyethylene alkyl dimethyl ammonium salt, a dipolyoxyethylene alkyl methyl ammonium salt, a tripolyoxyethylene alkyl ammonium salt, an alkyl benzyl dimethyl ammonium salt, an alkyl pyridinium salt, a monoalkyl amine salt, a monoalkylamide amine salt, and a cationic cellulose.

Illustrative examples of the nonionic surfactant include a polyoxyethylene alkyl ether, a polyoxyethylene polyoxypropylene alkyl ether, a polyoxyethylene alkylphenyl ether, a polyethylene glycol fatty acid ester, a sorbitan fatty acid ester, a polyoxyethylene sorbitan fatty acid ester, a polyoxyethylene sorbit fatty acid ester, a glycerin fatty acid ester, a polyoxyethylene glycerin fatty acid ester, a polyglycerin fatty acid ester, a propylene glycol fatty acid ester, a polyoxyethylene castor oil, a polyoxyethylene hard castor oil, a fatty acid ester of a polyoxyethylene hard castor oil, a polyoxyethylene alkyl amine, a polyoxyethylene fatty acid amide, a polyoxyalkylene-modified organopolysiloxane, an organopolysiloxane co-modified with a polyoxyalkylene and an alkyl, a polyglycerin-modified organopolysiloxane (Japanese Patent No. 2613124), an alkyl-modified silicone having a branched silicone (Japanese Patent No. 3850202), an organopolysiloxane co-modified with a branched silicone polyoxyalkylene and a long chain alkyl (Japanese Patent No. 3724988), and an organopolysiloxane modified with a silicone-branched polyglycerin (Japanese Patent No. 3976226).

Illustrative examples of the amphoteric surfactant include a betaine, an aminocarboxylic acid salt, an imidazoline derivative, and an amide amine type.

Among these surfactants, a linear or a branched organopolysiloxane having a polyglycerin chain or a polyoxyalkylene chain in the molecule, or an organopolysiloxane co-modified with an alkyl, with the amount of a hydrophilic polyoxyethylene group or a hydrophilic polyglycerin group being 10 to 70% by mass in the molecule, is preferable. In addition, amount thereof to be blended is preferably 0.1 to 20% by mass, or particularly preferably 0.2 to 10% by mass, relative to the totality of the cosmetic.

The silicone resin of the component (G) is the one, which is in the form of a gum or in a solid state at a normal temperature and is uniformly soluble in decamethyl cyclopentasiloxane.

The silicone resin in the form of a gum is preferably a linear polysiloxane shown by the following general formula (3).

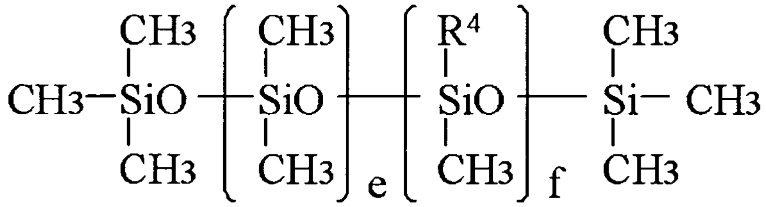

(3)

In the formula (3), $R^4$ is selected from a methyl group; an alkyl group having 6 to 20 carbon atoms; an amino-containing alkyl group, an fluorine-substituted alkyl group, and an alkyl group having a quaternary ammonium salt, the number of carbon atoms contained in these alkyl groups being 3 to 15; and the "e" is a number preferably in the range of 1000 to 20000, the "f" is a number preferably in the range of 1 to 5000, and e+f is a number preferably in the range of 2000 to 20000.

Illustrative examples of the silicone resin in the solid state include a silicone net-work compound with an arbitrary combination of a trialkylsiloxy unit (M unit), a dialkylsiloxy unit (D unit), a monoalkylsiloxy unit (T unit), and a four-functional siloxy unit (Q unit); namely a MQ resin, a MDQ resin, a MTQ resin, a MDTQ resin, a T resin, a TD resin, a TQ resin, a TDQ resin, and so on. Further included therein are an acryl silicone resin such as an acryl/silicone graft copolymer and an acryl/silicone block copolymer (specific examples thereof include KP-545: a cyclic organopolysiloxane solution of an acryl/silicone graft copolymer manufactured by Shin-Etsu Chemical Co., Ltd.). In addition, an acryl silicone resin having, within its molecular structure, at least one part selected from a pyrrolidone part, a long chain alkyl part, a polyoxyalkylene part, a fluoroalkyl part, an anionic part of a carboxylic acid and the like, may be used.

Silicone resins such as the silicone resin in the gum form, the silicone net-work compound, the acryl silicone resin, and so on may be used by dissolving them in a low-viscosity silicone oil, a volatile silicone resin, or other solvent. Amount of the silicone resin to be used is, as the resin component, preferably 0.1 to 20% by mass, or more preferably 1 to 10% by mass, relative to the totality of the cosmetic.

As to the paste composition of the component (H) comprising a crosslinking organopolysiloxane and a liquid oil, the both being in the public domain, other than the paste composition of the present invention, illustrative examples thereof include those described in Japanese Patent Publication (Kokoku) No. H06-55897, Japanese Patent No. 2631772, Japanese Patent No. 3969728, Japanese Patent No. 4001342, and Japanese Patent No. 4490817. Illustrative examples of the crosslinking organopolysiloxane swelled with a silicone oil include KSG-15, 16, 17, 18, 21, 24, 210, and 710 (manufactured by Shin-Etsu Chemical Co., Ltd.); illustrative examples of the crosslinking organopolysiloxane swelled with a hydrocarbon oil include KSG-31, 32, 34, 310, 320, 340, 41, 42, 44, 810, 820, and 840 (manufactured by Shin-Etsu Chemical Co., Ltd.); and illustrative examples of the crosslinking organopolysiloxane swelled with an ester oil include KSG-33, 330, 43, and 830 (manufactured by Shin-Etsu Chemical Co., Ltd.). The composition comprising a crosslinking organopolysiloxane and a liquid oil, the both being in the public domain, may be used with the amount thereof is preferably 0.1 to 50% by mass, or more preferably 1 to 30% by mass, relative to the totality of the cosmetic.

The cosmetic of the present invention may be added with a component generally used in a usual cosmetic; illustrative examples thereof include an oil-soluble gelation agent, an antiperspirant, a UV-absorber, a UV absorbing-scattering agent, a moisturizer, an antibacterial preservative, a fragrance, a salt, an antioxidant, a pH regulator, a chelating agent, an algefacient, an anti-inflammatory agent, a skin beautifying component (a skin-lightening agent, a cell activator, a rough-skin improver, a blood circulation acceletor, a skin astringent agent, an antiseborrheic agent, and so on), a vitamin, an amino acid, a nucleic acid, a hormone, a clathrate compound, and a hair-immobilizing agent.

Illustrative examples of the oil-soluble gelling agent include a metal soap such as aluminum stearate, magnesium stearate, and zinc myristate; an amino acid derivative such as N-lauroyl-L-glutamic acid and $\alpha,\gamma$-di-n-butyl amine; a dextrin fatty acid ester such as dextrin palmitate ester, dextrin stearate ester, and dextrin 2-ethylhexanoate palmitate ester; a sucrose fatty acid ester such as sucrose palmitate ester and sucrose stearate ester; a fructo-oligosaccharide fatty acid ester such as fructo-oligosaccharide stearate ester and fructo-oligosaccharide 2-ethylhexanoate ester; a benzylidene derivative of sorbitol such as monobenzylidene sorbitol and dibenzylidene sorbitol; an organic-modified clay mineral such as dimethyl benzyl dodecyl ammonium montomorillonite clay and dimethyl dioctadecyl ammonium montomorillonite clay.

Illustrative examples of the antiperspirant include aluminum chlorohydrate, aluminum chloride, aluminum sesquichlorohydrate, zirconyl hydroxy chloride, aluminum zirconium hydroxy chloride, and aluminum zirconium glycine complex.

Illustrative examples of the UV-absorber include a benzoic acid UV-absorber such as para-amino benzoic acid; an anthranilic acid UV-absorber such as methyl anthranilate; a salicylic acid UV-absorber such as methyl salicylate, octyl salicylate, and trimethylcyclohexyl salicylate; a cinnamic acid UV-absorber such as octyl para-methoxy cinnamate; a benzophenone UV-absorber such as 2,4-dihydroxybenzophenone; an urocanic acid UV-absorber such as ethyl urocanate; a dibenzoylmethane UV-absorber such as 4-t-butyl-4'-methoxydibenzoylmethane; phenyl benzimidazole sulfonic

acid; and a triazine derivative.

Illustrative examples of the UV absorbing-scattering agent include a particle, which absorbs and scatters a UV-beam, such as a titanium oxide microparticle, a titanium oxide microparticle containing an iron, a zinc oxide microparticle, a cerium oxide microparticle, and a composite material of them. A dispersed material obtained by dispersing the particle, which absorbs and scatters a UV-beam into an oil prior to use may also be used.

Illustrative examples of the moisturizer include glycerin, sorbitol, propylene glycol, dipropylene glycol, 1,3-butylene glycol, pentylene glycol, glucose, xylitol, maltitol, polyethylene glycol, hyaluronic acid, chondroitin sulfate, pyrrolidone carboxylate salt, polyoxyethylene methyl glucoside, polyoxypropylene methyl glucoside, egg yolk lecithin, soybean lecithin, phosphatidyl choline, phosphatidyl ethanolamine, phosphatidyl serine, phosphatidyl glycerol, phosphatidyl inositol, and sphingo phospholipid.

Illustrative examples of the antibacterial preservative include a para-oxybenzoate alkyl ester, benzoic acid, sodium benzoate, sorbic acid, potassium sorbate, and phenoxy ethanol. Illustrative examples of the antibacterial agent include benzoic acid, salicylic acid, carbolic acid, sorbic acid, a para-oxybenzoic acid alkyl ester, p-chloro-m-cresol, hexachlorophene, benzalkonium chloride, chlorhexidine chloride, trichlorocarbanilide, a photosensitive element, phenoxy ethanol, and methyl isothiazoline.

Illustrative examples of the fragrance include a natural fragrance and a synthetic fragrance. Illustrative examples of the natural fragrance include a plant fragrance separated from a flower, a leaf, a trunk, and a fruit skin; and an animal fragrance such as musk and civet. Illustrative examples of the synthetic fragrance include a hydrocarbon such as monoterpene; an alcohol such as an aliphatic alcohol and an aromatic alcohol; an aldehyde such as a terpene aldehyde and an aromatic aldehyde; a ketone such as an alicyclic ketone; an ester such as a terpene ester; a lactone; a phenol; an oxide; a nitrogen-containing compound; and an acetal.

As to the salt, an inorganic salt, an organic salt, an amine salt, and an amino acid salt may be mentioned. Illustrative examples of the inorganic salt include a sodium, a potassium, a magnesium, a calcium an aluminum, a zirconium, and a zinc salt of an inorganic acid such as hydrochloric acid, sulfuric acid, carbonic acid, and nitric acid. Illustrative examples of the organic salt include a salt of an organic acid such as acetic acid, dehydroacetic acid, citric acid, malic acid, succinic acid, ascorbic acid, and stearic acid. Illustrative examples of the amine salt include a salt of amine such as triethanol amine. Illustrative examples of the amino acid salt include a salt of an amino acid such as a glutamic acid. In addition, a salt of hyaluronic acid and chondroitin sulfate, an aluminum zirconium glycine complex, and a neutralized salt obtained by neutralization of an acid and an alkali used in a cosmetic prescription may be used.

Illustrative examples of the antioxidant include tocopherol, p-t-butylphenol, butyl hydroxy anisole, dibutyl hydroxy toluene, and phytic acid.

Illustrative examples of the pH-regulator include lactic acid, citric acid, glycolic acid, succinic acid, tartaric acid, dl-malic acid, potassium carbonate, sodium hydrogencarbonate, and ammonium hydrogencarbonate.

Illustrative examples of the chelating agent include alanine, sodium edetate, sodium polyphosphate, sodium metaphosphate, and phosphoric acid. Illustrative examples of the algefacient include L-menthol and camphor. Illustrative examples of the anti-inflammatory agent include allantoin, glycyrrhizic acid and its salt, glycyrrhetic acid and stearyl glycyrrhetinate, tranexamic acid, and azulene.

Illustrative examples of the skin-beautifying component include a skin-lightening agent such as a placenta extract, arbutin, glutathione, and a strawberry geranium; a cell activator such as a royal jelly, a photosensitive element, a cholesterol derivative, and an extract from hemolysed blood of young calves; a rough-skin improver; a blood circulation promoter such as nonylic acid warenylamide, benzyl nicotinate ester, $\beta$-butoxyethyl nicotinate ester, capsaicin, zingerone, cantharides tincture, ichthammol, caffeine, tannic acid, $\alpha$-borneol, nicotinic acid tocopherol, inositol hexanicotinate, cyclandelate, cinnarizine, tolazoline, acetyl choline, verapamil, cepharanthin, and $\gamma$-orizanol; a skin astringent agent such as zinc oxide and tannic acid; and an antiseborrheic agent such as sulfur and thianthrol.

Illustrative examples of the vitamin include a vitamin A such as a vitamin A oil, retinol, retinol acetate, and retinol palmitate; a vitamin B including a vitamin $B_2$ such as riboflavin, riboflavin butyrate, and a flavin adenine nucleotide, a vitamin $B_6$ such as pyridoxine hydrochloride salt, pyridoxine dioctanoate, and pyridoxine tripalmitate, a vitamin $B_{12}$ and its derivative, and vitamin $B_{15}$ and its derivative; a vitamin C such as L-ascorbic acid, L-ascorbic acid dipalmitate ester, sodium L-ascorbic-2-sulfate, and dipotassium L-ascorbic acid phosphate diester; a vitamin D such as ergocalciferol and cholecalciferol; a vitamin E such as $\alpha$-tocopherol, $\beta$-tocopherol, $\gamma$-tocopherol, dl-$\alpha$-tocopherol acetate, dl-$\alpha$-tocopherol nicotinate, and dl-$\alpha$-tocopherol succinate; a nicotinic acid such as nicotinic acid, benzyl nicotinate, and a nicotinic acid amide; vitamin H; vitamin P; a pantothenic acid such as calcium pantothenate, D-pantothenyl alcohol, pantothenyl ethyl ether, and acetyl pantothenyl ethyl ether; and biotin.

Illustrative examples of the amino acid include glycine, valine, leucine, isoleucine, serine, threonine, phenylalanine, arginine, lysine, aspartic acid, glutamic acid, cystine, cysteine, methionine, and tryptophan.

Illustrative examples of the nucleic acid include deoxyribonucleic acid. Illustrative examples of the hormone include estradiol and ethenyl estradiol. Illustrative examples of the clathrate compound include cyclodextrin.

As to the hair-immobilizing agent, an amphoteric polymer, an anionic polymer, a cationic polymer, and a nonionic

polymer may be mentioned; and illustrative examples of the hair-immobilizing agent include a polyvinyl pyrrolidone polymer such as polyvinyl pyrrolidone and vinyl pyrrolidone/vinyl acetate copolymer; an acidic vinyl ether polymer such as methyl vinyl ether/maleic anhydride alkyl half-ester copolymer; an acidic polyvinyl acetate polymer such as vinyl acetate/crotonic acid copolymer; an acidic acryl polymer such as a (meth)acrylic acid/alkyl (meth)acrylate copolymer and a (meth)acrylic acid/alkyl (meth)acrylate/alkyl acrylamide copolymer; and an amphoteric acryl polymer such as a N-methacryloylethyl-N,N-dimethyl ammonium/α-N-methylcarboxybetaine/alkyl (meth)acrylate copolymer and hydroxypropyl (meth)acrylate/butylaminoethyl methacrylate/acrylic acid octyl amide copolymer. In addition, a naturally-occuring polymer such as cellulose or its derivative, keratin or its derivative, and collagen or its derivative may be used suitably.

The cosmetics of the present invention are those blended with the foregoing cosmetic components, and include a skin care cosmetic such as a milky lotion, a cream, a cleansing cream, a pack, an oil liquid, a massage material, an essence, a cleaning cosmetic, a deodorant, a hand cream, and a lip cream; a make-up cosmetic such as a make-up foundation, a white powder, a liquid foundation, an oil foundation, a rouge, an eye shadow, a mascara, an eye liner, an eye blow, and a lipstick; a hair cosmetic such as a shampoo, a rinse, a conditioner, a treatment, and a setting material; an antiperspirant; and a UV-protective cosmetic such as a sunscreen lotion and a sun-cut cream. These cosmetics may be in any form of a liquid, a lotion, a cream, a solid, a paste, a gel, a powder, pressed, multi-layered, a mousse, a spray, a stick, and so on.

EXAMPLES

Hereinafter, the present invention will be explained in more detail by showing Synthesis Examples, Comparative Synthesis Examples, and Examples; but the present invention is not at all limited to them. Meanwhile, a dynamic viscosity was measured at 25°C by using an Ostwald viscometer, and a viscosity was measured at 25°C by using a rotational viscometer DV-II+ (manufactured by Brookfield Engineering Laboratories, Inc.).

[Synthesis Example 1]

In to an emulsifying vessel were added 200.0 parts by mass of the methyl hydrogen polysiloxane shown by the following average composition formula (4) and 96.0 parts by mass of the methyl vinyl polysiloxane shown by the following average composition formula (5); and then, they were mixed by using a homomixer with stirring at 2000 rpm. Into the resulting mixture were added 0.7 parts by mass of polyoxyethylene lauryl ether (added mole of ethyleneoxide being 9) and 60 parts by mass of water; and then, they were stirred by using a homomixer at 6000 rpm for 15 minutes. Thereafter, 220 parts by mass of water was added into this mixture with stirring thereof at 2000 rpm to obtain a white and homogeneous emulsion.

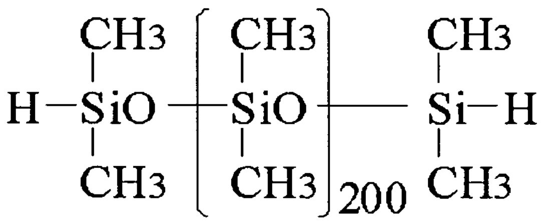

(4)

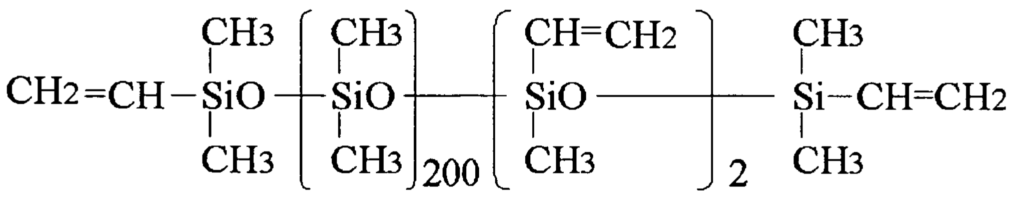

(5)

Then, this emulsion was charged into a reactor equipped with an anchor agitator; and after the temperature thereof was made 15 to 20°C, the mixture solution of 0.46 parts by mass of the toluene solution containing 0.5% by mass of chloroplatinic acid, 0.87 parts by mass of polyoxyethylene lauryl ether (added mole of ethyleneoxide being 4), and 0.87 parts by mass of polyoxyethylene lauryl ether (added mole of ethyleneoxide being 23) was added into the emulsion with agitating the emulsion, and then they were agitated for 12 hours with keeping the temperature thereof at 15 to 20°C to obtain an aqueous disperse solution of organopolysiloxane elastomer microparticles. The form of this organopolysiloxane elastomer microparticle was confirmed spherical by an optical microscope; and the volume-average

particle diameter thereof was 12 μm as measured by a measurement instrument for particle diameter distribution (Multisizer 3, manufactured by Beckman Coulter, Inc.).

The obtained aqueous disperse solution of the organopolysiloxane elastomer microparticles was filtrated by using a filter press; and then, the solid material thus separated was transferred to a tray made of a stainless steel, dried at 105°C by using a dryer of a hot-air circulation type, and then the dried mass was crushed by a jet mill to obtain free-flow microparticles. The form of this microparticle was spherical by observation with an electron microscope; and the volume-average particle diameter thereof after dispersed into water containing a surfactant was 12 μm as measured by the before-mentioned Multisizer 3. This organopolysiloxane elastomer microparticle was designated as "Elastomer Microparticle A".

Into a glass bottle were taken 5.0 g of Elastomer Microparticle A and 50 g of the methyl polysiloxane shown in Table 1; and then, after the bottle was shaken for 30 minutes, it was allowed to stand at room temperature for 3 days. Thereafter, a solid portion was separated by a filter press, and then a mass of the solid cake was measured to obtain an oil-absorption amount thereof by the following equation.

The oil-absorption amount was obtained similarly by using 50 g of the decamethyl pentasiloxane shown in Table 1 in place of 50 g of the methyl polysiloxane.

Each result thereof is shown in Table 1.

$$\text{Oil-absorption amount (g)} = [\text{amount of solid cake (g)}] - 5.0\ \text{(g)}$$

Further, 100 parts by mass of Elastomer Microparticle A and 300 parts by mass of the dimethyl polysiloxane (dynamic viscosity of 6 $mm^2$/second) were mixed and then fully kneaded by a three-roll mill to obtain a paste composition having the viscosity of 823,000 mPa·s at the ratio of the organopolysiloxane elastomer microparticle to the dimethyl polysiloxane being 25/75 by mass. This paste composition was designated as "Paste Composition A".

[Synthesis Example 2]

In to an emulsifying vessel were added 200.0 parts by mass of the methyl hydrogen polysiloxane shown by the average composition formula (4) and 96.0 parts by mass of the methyl vinyl polysiloxane shown by the average composition formula (5); and then, they were mixed by using a homomixer with stirring at 2000 rpm. Into the resulting mixture were added 0.46 parts by mass of polyoxyethylene lauryl ether (added mole of ethyleneoxide being 4), 0.58 parts by mass of polyoxyethylene lauryl ether (added mole of ethyleneoxide being 23), and 50 parts by mass of water; and then, they were stirred by using a homomixer at 6000 rpm for 15 minutes. Thereafter, 230 parts by mass of water was added into this mixture with stirring thereof at 2000 rpm to obtain a white and homogeneous emulsion.

Then, this emulsion was charged into a reactor equipped with an anchor agitator; and after the temperature thereof was made 15 to 20°C, the mixture solution of 0.46 parts by mass of the toluene solution containing 0.5% by mass of chloroplatinic acid, 0.70 parts by mass of polyoxyethylene lauryl ether (added mole of ethyleneoxide being 4), and 0.70 parts by mass of polyoxyethylene lauryl ether (added mole of ethyleneoxide being 23) was added into the emulsion with agitating the emulsion, and then they were agitated for 12 hours with keeping the temperature thereof at 15 to 20°C to obtain an aqueous disperse solution of organopolysiloxane elastomer microparticles. The form of this organopolysiloxane elastomer microparticle was confirmed spherical by an optical microscope; and the volume-average particle diameter thereof was 5 μm as measured by the before-mentioned Multisizer 3.

The obtained aqueous disperse solution of the organopolysiloxane elastomer microparticles was filtrated by using a filter press; and then, the solid material thus separated was transferred to a tray made of a stainless steel, dried at 105°C by using a dryer of a hot-air circulation type, and then the dried mass was crushed by a jet mill to obtain free-flow microparticles. The form of this microparticle was spherical by observation with an electron microscope; and the volume-average particle diameter thereof after dispersed into water containing a surfactant was 5 μm as measured by the before-mentioned Multisizer 3. This organopolysiloxane elastomer microparticle was designated as "Elastomer Microparticle B".

Into a glass bottle were taken 5.0 g of Elastomer Microparticle B and 50 g of the methyl polysiloxane shown in Table 1; and then, after the bottle was shaken for 30 minutes, it was allowed to stand at room temperature for 3 days. Thereafter, a solid portion was separated by a filter press, and then a mass of the solid cake was measured to obtain an oil-absorption amount thereof by the equation shown in Synthesis Example 1.

The oil-absorption amount was obtained similarly by using 50 g of the decamethyl pentasiloxane shown in Table 1 in place of 50 g of the methyl polysiloxane.

Each result thereof is shown in Table 1.

Further, 100 parts by mass of Elastomer Microparticle B and 300 parts by mass of the dimethyl polysiloxane (dynamic viscosity of 6 $mm^2$/second) were mixed and then fully kneaded by a three-roll mill to obtain a paste composition having the viscosity of 861,000 mPa·s at the ratio of the organopolysiloxane elastomer microparticle to the dimethyl polysiloxane being 25/75 by mass. This paste composition was designated as "Paste Composition B".

[Synthesis Example 3]

In to an emulsifying vessel were added 200.0 parts by mass of the methyl hydrogen polysiloxane shown by the average composition formula (4) and 96.0 parts by mass of the methyl vinyl polysiloxane shown by the average composition formula (5); and then, they were mixed by using a homomixer with stirring at 2000 rpm. Into the resulting mixture were added 0.48 parts by mass of polyoxyethylene lauryl ether (added mole of ethyleneoxide being 4), 0.36 parts by mass of polyoxyethylene lauryl ether (added mole of ethyleneoxide being 23), and 80 parts by mass of water; and then, they were stirred by using a homomixer at 4500 rpm for 15 minutes. Thereafter, 200 parts by mass of water was added into this mixture with stirring thereof at 2000 rpm to obtain a white and homogeneous emulsion.

Then, this emulsion was charged into a reactor equipped with an anchor agitator; and after the temperature thereof was made 15 to 20°C, the mixture solution of 0.46 parts by mass of the toluene solution containing 0.5% by mass of chloroplatinic acid, 0.80 parts by mass of polyoxyethylene lauryl ether (added mole of ethyleneoxide being 4), and 0.80 parts by mass of polyoxyethylene lauryl ether (added mole of ethyleneoxide being 23) was added into the emulsion with agitating the emulsion, and then they were agitated for 12 hours with keeping the temperature thereof at 15 to 20°C to obtain an aqueous disperse solution of organopolysiloxane elastomer microparticles. The form of this organopolysiloxane elastomer microparticle was confirmed spherical by an optical microscope; and the volume-average particle diameter thereof was 28 μm as measured by the before-mentioned Multisizer 3.

The obtained aqueous disperse solution of the organopolysiloxane elastomer microparticles was filtrated by using a filter press; and then, the solid material thus separated was transferred to a tray made of a stainless steel, dried at 105°C by using a dryer of a hot-air circulation type, and then the dried mass was crushed by a jet mill to obtain free-flow microparticles. The form of this microparticle was spherical by observation with an electron microscope; and the volume-average particle diameter thereof after dispersed into water containing a surfactant was 28 μm as measured by the before-mentioned Multisizer 3. This organopolysiloxane elastomer microparticle was designated as "Elastomer Microparticle C".

Into a glass bottle were taken 5.0 g of Elastomer Microparticle C and 50 g of the methyl polysiloxane shown in Table 1; and then, after the bottle was shaken for 30 minutes, it was allowed to stand at room temperature for 3 days. Thereafter, a solid portion was separated by a filter press, and then a mass of the solid cake was measured to obtain an oil-absorption amount thereof by the equation shown in Synthesis Example 1.

The oil-absorption amount was obtained similarly by using 50 g of the decamethyl pentasiloxane shown in Table 1 in place of 50 g of the methyl polysiloxane.

Each result thereof is shown in Table 1.

Further, 100 parts by mass of Elastomer Microparticle C and 300 parts by mass of the dimethyl polysiloxane (dynamic viscosity of 6 $mm^2$/second) were mixed and then fully kneaded by a three-roll mill to obtain a paste composition having the viscosity of 616,000 mPa·s at the ratio of the organopolysiloxane elastomer microparticle to the dimethyl polysiloxane being 25/75 by mass. This paste composition was designated as "Paste Composition C".

[Synthesis Example 4]

In to an emulsifying vessel were added 145.0 parts by mass of the methyl hydrogen polysiloxane shown by the following average composition formula (6) and 155.0 parts by mass of the methyl vinyl polysiloxane shown by the following average composition formula (7); and then, they were mixed by using a homomixer with stirring at 2000 rpm. Into the resulting mixture were added 0.7 parts by mass of polyoxyethylene lauryl ether (added mole of ethyleneoxide being 9) and 60 parts by mass of water; and then, they were stirred by using a homomixer at 6000 rpm for 15 minutes. Thereafter, 220 parts by mass of water was added into this mixture with stirring thereof at 2000 rpm to obtain a white and homogeneous emulsion.

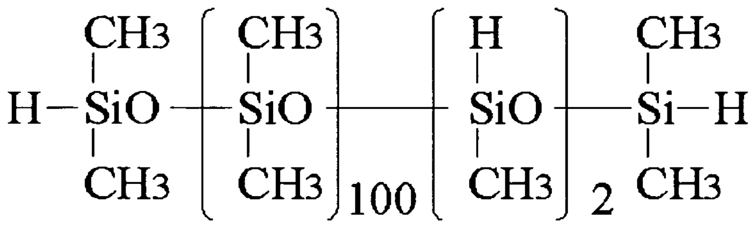

(6)

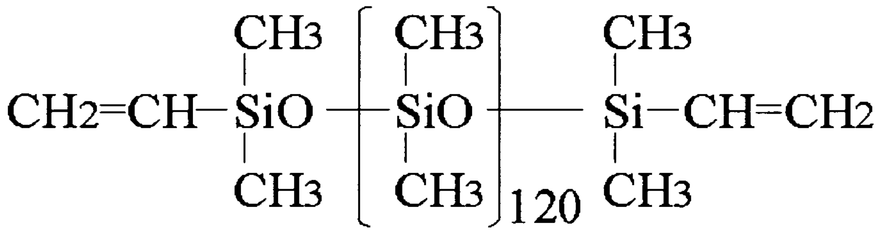

(7)

Then, this emulsion was charged into a reactor equipped with an anchor agitator; and after the temperature thereof was made 15 to 20°C, the mixture solution of 0.46 parts by mass of the toluene solution containing 0.5% by mass of chloroplatinic acid, 0.87 parts by mass of polyoxyethylene lauryl ether (added mole of ethyleneoxide being 4), and 0.87 parts by mass of polyoxyethylene lauryl ether (added mole of ethyleneoxide being 23) was added into the emulsion with agitating the emulsion, and then they were agitated for 12 hours with keeping the temperature thereof at 15 to 20°C to obtain an aqueous disperse solution of organopolysiloxane elastomer microparticles. The form of this organopolysiloxane elastomer microparticle was confirmed spherical by an optical microscope; and the volume-average particle diameter thereof was 10 μm as measured by the before-mentioned Multisizer 3.

The obtained aqueous disperse solution of the organopolysiloxane elastomer microparticles was filtrated by using a filter press; and then, the solid material thus separated was transferred to a tray made of a stainless steel, dried at 105°C by using a dryer of a hot-air circulation type, and then the dried mass was crushed by a jet mill to obtain free-flow microparticles. The form of this microparticle was spherical by observation with an electron microscope; and the volume-average particle diameter thereof after dispersed into water containing a surfactant was 10 μm as measured by the before-mentioned Multisizer 3. This organopolysiloxane elastomer microparticle was designated as "Elastomer Microparticle D".

Into a glass bottle were taken 5.0 g of Elastomer Microparticle D and 50 g of the methyl polysiloxane shown in Table 1; and then, after the bottle was shaken for 30 minutes, it was allowed to stand at room temperature for 3 days. Thereafter, a solid portion was separated by a filter press, and then a mass of the solid cake was measured to obtain an oil-absorption amount thereof by the equation shown in Synthesis Example 1.

The oil-absorption amount was obtained similarly by using 50 g of the decamethyl pentasiloxane shown in Table 1 in place of 50 g of the methyl polysiloxane.

Each result thereof is shown in Table 1.

Further, 100 parts by mass of Elastomer Microparticle D and 270 parts by mass of the dimethyl polysiloxane (dynamic viscosity of 6 $mm^2$/second) were mixed and then fully kneaded by a three-roll mill to obtain a paste composition having the viscosity of 683,000 mPa·s at the ratio of the organopolysiloxane elastomer microparticle to the dimethyl polysiloxane being 27/73 by mass. This paste composition was designated as "Paste Composition D".

[Synthesis Example 5]

In to an emulsifying vessel were added 105.0 parts by mass of the methyl hydrogen polysiloxane shown by the following average composition formula (8) and 195.0 parts by mass of the methyl vinyl polysiloxane shown by the following average composition formula (9); and then, they were mixed by using a homomixer with stirring at 2000 rpm. Into the resulting mixture were added 0.7 parts by mass of polyoxyethylene lauryl ether (added mole of ethyleneoxide being 9) and 60 parts by mass of water; and then, they were stirred by using a homomixer at 6000 rpm for 15 minutes. Thereafter, 220 parts by mass of water was added into this mixture with stirring thereof at 2000 rpm to obtain a white and homogeneous emulsion.

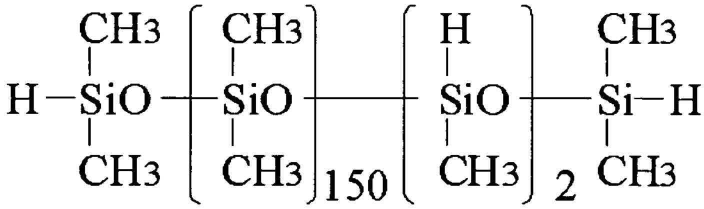 (8)

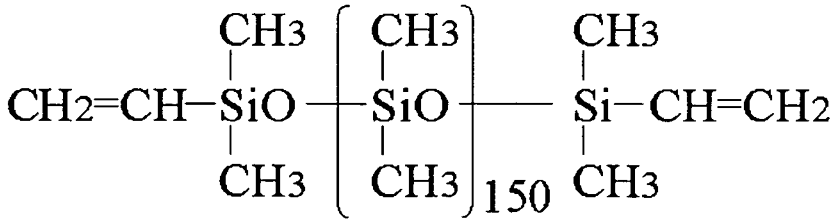 (9)

Then, this emulsion was charged into a reactor equipped with an anchor agitator; and after the temperature thereof was made 15 to 20°C, the mixture solution of 0.46 parts by mass of the toluene solution containing 0.5% by mass of chloroplatinic acid, 0.87 parts by mass of polyoxyethylene lauryl ether (added mole of ethyleneoxide being 4), and 0.87 parts by mass of polyoxyethylene lauryl ether (added mole of ethyleneoxide being 23) was added into the emulsion with agitating the emulsion, and then they were agitated for 12 hours with keeping the temperature thereof at 15 to 20°C to obtain an aqueous disperse solution of organopolysiloxane elastomer microparticles. The form of this organopolysiloxane elastomer microparticle was confirmed spherical by an optical microscope; and the volume-average particle diameter thereof was 12 μm as measured by the before-mentioned Multisizer 3.

The obtained aqueous disperse solution of the organopolysiloxane elastomer microparticles was filtrated by using a filter press; and then, the solid material thus separated was transferred to a tray made of a stainless steel, dried at 105°C by using a dryer of a hot-air circulation type, and then the dried mass was crushed by a jet mill to obtain free-flow microparticles. The form of this microparticle was spherical by observation with an electron microscope; and the volume-average particle diameter thereof after dispersed into water containing a surfactant was 12 μm as measured by the before-mentioned Multisizer 3. This organopolysiloxane elastomer microparticle was designated as "Elastomer Microparticle E".

Into a glass bottle were taken 5.0 g of Elastomer Microparticle E and 50 g of the methyl polysiloxane shown in Table 1; and then, after the bottle was shaken for 30 minutes, it was allowed to stand at room temperature for 3 days. Thereafter, a solid portion was separated by a filter press, and then a mass of the solid cake was measured to obtain an oil-absorption amount thereof by the equation shown in Synthesis Example 1.

The oil-absorption amount was obtained similarly by using 50 g of the decamethyl pentasiloxane shown in Table 1 in place of 50 g of the methyl polysiloxane.

Each result thereof is shown in Table 1.

Further, 100 parts by mass of Elastomer Microparticle E and 300 parts by mass of the dimethyl polysiloxane (dynamic viscosity of 6 $mm^2$/second) were mixed and then fully kneaded by a three-roll mill to obtain a paste composition having the viscosity of 858,000 mPa·s at the ratio of the organopolysiloxane elastomer microparticle to the dimethyl polysiloxane being 25/75 by mass. This paste composition was designated as "Paste Composition E".

[Synthesis Example 6]

In to an emulsifying vessel were added 105.0 parts by mass of the methyl hydrogen polysiloxane shown by the average composition formula (8) and 195.0 parts by mass of the methyl vinyl polysiloxane shown by the average composition formula (9); and then, they were mixed by using a homomixer with stirring at 2000 rpm. Into the resulting mixture were added 0.48 parts by mass of polyoxyethylene lauryl ether (added mole of ethyleneoxide being 4), 0.36 parts by mass of polyoxyethylene lauryl ether (added mole of ethyleneoxide being 23), and 80 parts by mass of water; and then, they were stirred by using a homomixer at 4500 rpm for 15 minutes. Thereafter, 200 parts by mass of water was added into this mixture with stirring thereof at 2000 rpm to obtain a white and homogeneous emulsion.

Then, this emulsion was charged into a reactor equipped with an anchor agitator; and after the temperature thereof was made 15 to 20°C, the mixture solution of 0.46 parts by mass of the toluene solution containing 0.5% by mass of chloroplatinic acid, 0.80 parts by mass of polyoxyethylene lauryl ether (added mole of ethyleneoxide being 4), and 0.80 parts by mass of polyoxyethylene lauryl ether (added mole of ethyleneoxide being 23) was added into the emulsion with agitating the emulsion, and then they were agitated for 12 hours with keeping the temperature thereof at

15 to 20°C to obtain an aqueous disperse solution of organopolysiloxane elastomer microparticles. The form of this organopolysiloxane elastomer microparticle was confirmed spherical by an optical microscope; and the volume-average particle diameter thereof was 30 μm as measured by the before-mentioned Multisizer 3.

The obtained aqueous disperse solution of the organopolysiloxane elastomer microparticles was filtrated by using a filter press; and then, the solid material thus separated was transferred to a tray made of a stainless steel, dried at 105°C by using a dryer of a hot-air circulation type, and then the dried mass was crushed by a jet mill to obtain free-flow microparticles. The form of this microparticle was spherical by observation with an electron microscope; and the volume-average particle diameter thereof after dispersed into water containing a surfactant was 30 μm as measured by the before-mentioned Multisizer 3. This organopolysiloxane elastomer microparticle was designated as "Elastomer Microparticle F".

Into a glass bottle were taken 5.0 g of Elastomer Microparticle F and 50 g of the methyl polysiloxane shown in Table 1; and then, after the bottle was shaken for 30 minutes, it was allowed to stand at room temperature for 3 days. Thereafter, a solid portion was separated by a filter press, and then a mass of the solid cake was measured to obtain an oil-absorption amount thereof by the equation shown in Synthesis Example 1.

The oil-absorption amount was obtained similarly by using 50 g of the decamethyl pentasiloxane shown in Table 1 in place of 50 g of the methyl polysiloxane.

Each result thereof is shown in Table 1.

Further, 100 parts by mass of Elastomer Microparticle F and 300 parts by mass of the dimethyl polysiloxane (dynamic viscosity of 6 $mm^2$/second) were mixed and then fully kneaded by a three-roll mill to obtain a paste composition having the viscosity of 676,000 mPa·s at the ratio of the organopolysiloxane elastomer microparticle to the dimethyl polysiloxane being 25/75 by mass. This paste composition was designated as "Paste Composition F".

[Synthesis Example 7]

In to an emulsifying vessel were added 133.0 parts by mass of the methyl hydrogen polysiloxane shown by the following average composition formula (10) and 167.0 parts by mass of the methyl vinyl polysiloxane shown by the following average composition formula (11); and then, they were mixed by using a homomixer with stirring at 2000 rpm. Into the resulting mixture were added 0.48 parts by mass of polyoxyethylene lauryl ether (added mole of ethyleneoxide being 4), 0.36 parts by mass of polyoxyethylene lauryl ether (added mole of ethyleneoxide being 23), and 70 parts by mass of water; and then, they were stirred by using a homomixer at 6000 rpm for 15 minutes. Thereafter, 210 parts by mass of water was added into this mixture with stirring thereof at 2000 rpm to obtain a white and homogeneous emulsion.

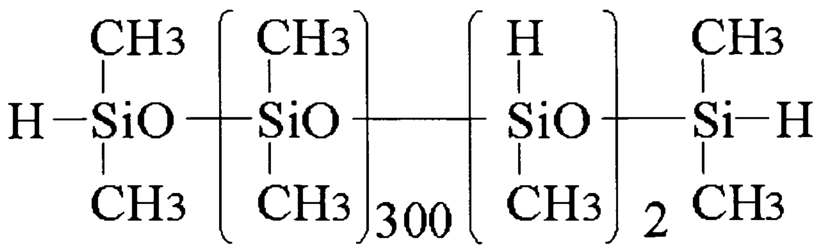

(10)

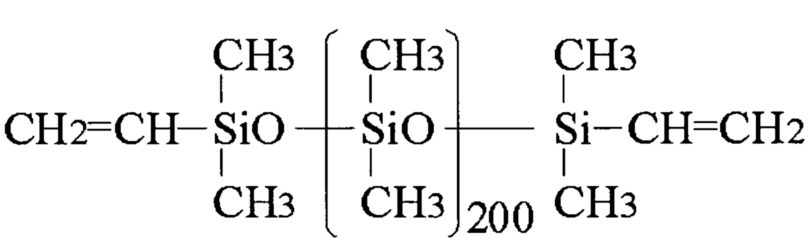

(11)

Then, this emulsion was charged into a reactor equipped with an anchor agitator; and after the temperature thereof was made 15 to 20°C, the mixture solution of 0.46 parts by mass of the toluene solution containing 0.5% by mass of chloroplatinic acid, 0.80 parts by mass of polyoxyethylene lauryl ether (added mole of ethyleneoxide being 4), and 0.80 parts by mass of polyoxyethylene lauryl ether (added mole of ethyleneoxide being 23) was added into the emulsion with agitating the emulsion, and then they were agitated for 12 hours with keeping the temperature thereof at 15 to 20°C to obtain an aqueous disperse solution of organopolysiloxane elastomer microparticles. The form of this organopolysiloxane elastomer microparticle was confirmed spherical by an optical microscope; and the volume-average particle diameter thereof was 18 μm as measured by the before-mentioned Multisizer 3.

The obtained aqueous disperse solution of the organopolysiloxane elastomer microparticles was filtrated by

using a filter press; and then, the solid material thus separated was transferred to a tray made of a stainless steel, dried at 105°C by using a dryer of a hot-air circulation type, and then the dried mass was crushed by a jet mill to obtain free-flow microparticles. The form of this microparticle was spherical by observation with an electron microscope; and the volume-average particle diameter thereof after dispersed into water containing a surfactant was 18 μm as measured by the before-mentioned Multisizer 3. This organopolysiloxane elastomer microparticle was designated as "Elastomer Microparticle G".

Into a glass bottle were taken 5.0 g of Elastomer Microparticle G and 50 g of the methyl polysiloxane shown in Table 1; and then, after the bottle was shaken for 30 minutes, it was allowed to stand at room temperature for 3 days. Thereafter, a solid portion was separated by a filter press, and then a mass of the solid cake was measured to obtain an oil-absorption amount thereof by the equation shown in Synthesis Example 1.

The oil-absorption amount was obtained similarly by using 50 g of the decamethyl pentasiloxane shown in Table 1 in place of 50 g of the methyl polysiloxane.

Each result thereof is shown in Table 1.

Further, 100 parts by mass of Elastomer Microparticle G and 300 parts by mass of the dimethyl polysiloxane (dynamic viscosity of 6 $mm^2$/second) were mixed and then fully kneaded by a three-roll mill to obtain a paste composition having the viscosity of 751,000 mPa·s at the ratio of the organopolysiloxane elastomer microparticle to the dimethyl polysiloxane being 25/75 by mass. This paste composition was designated as "Paste Composition G".

[Synthesis Example 8]

In to an emulsifying vessel were added 66.0 parts by mass of the methyl hydrogen polysiloxane shown by the following average composition formula (12) and 234.0 parts by mass of the methyl vinyl polysiloxane shown by the following average composition formula (13); and then, they were mixed by using a homomixer with stirring at 2000 rpm. Into the resulting mixture were added 0.7 parts by mass of polyoxyethylene lauryl ether (added mole of ethyleneoxide being 9) and 60 parts by mass of water; and then, they were stirred by using a homomixer at 6000 rpm for 15 minutes. Thereafter, 220 parts by mass of water was added into this mixture with stirring thereof at 2000 rpm to obtain a white and homogeneous emulsion.

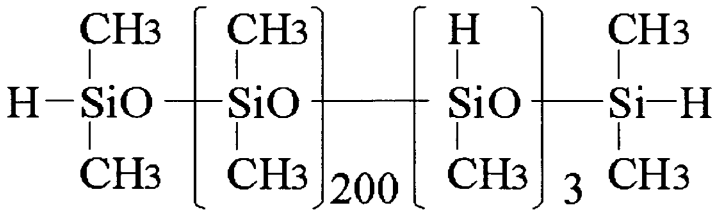

(12)

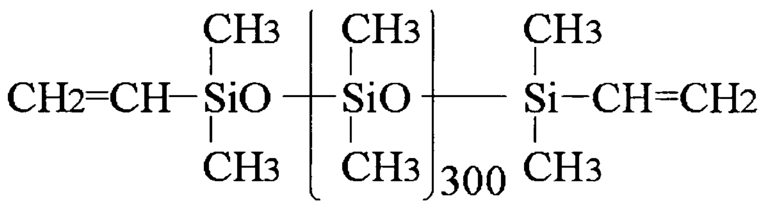

(13)

Then, this emulsion was charged into a reactor equipped with an anchor agitator; and after the temperature thereof was made 15 to 20°C, the mixture solution of 0.46 parts by mass of the toluene solution containing 0.5% by mass of chloroplatinic acid, 0.87 parts by mass of polyoxyethylene lauryl ether (added mole of ethyleneoxide being 4), and 0.87 parts by mass of polyoxyethylene lauryl ether (added mole of ethyleneoxide being 23) was added into the emulsion with agitating the emulsion, and then they were agitated for 12 hours with keeping the temperature thereof at 15 to 20°C to obtain an aqueous disperse solution of organopolysiloxane elastomer microparticles. The form of this organopolysiloxane elastomer microparticle was confirmed spherical by an optical microscope; and the volume-average particle diameter thereof was 13 μm as measured by the before-mentioned Multisizer 3.

The obtained aqueous disperse solution of the organopolysiloxane elastomer microparticles was filtrated by using a filter press; and then, the solid material thus separated was transferred to a tray made of a stainless steel, dried at 105°C by using a dryer of a hot-air circulation type, and then the dried mass was crushed by a jet mill to obtain free-flow microparticles. The form of this microparticle was spherical by observation with an electron microscope; and the volume-average particle diameter thereof after dispersed into water containing a surfactant was 13 μm as measured by the before-mentioned Multisizer 3. This organopolysiloxane elastomer microparticle was designated as "Elastomer Microparticle H".

Into a glass bottle were taken 5.0 g of Elastomer Microparticle H and 50 g of the methyl polysiloxane shown in Table 1; and then, after the bottle was shaken for 30 minutes, it was allowed to stand at room temperature for 3 days. Thereafter, a solid portion was separated by a filter press, and then a mass of the solid cake was measured to obtain an oil-absorption amount thereof by the equation shown in Synthesis Example 1.

The oil-absorption amount was obtained similarly by using 50 g of the decamethyl pentasiloxane shown in Table 1 in place of 50 g of the methyl polysiloxane.

Each result thereof is shown in Table 1.

Further, 100 parts by mass of Elastomer Microparticle H and 300 parts by mass of the dimethyl polysiloxane (dynamic viscosity of 6 $mm^2$/second) were mixed and then fully kneaded by a three-roll mill to obtain a paste composition having the viscosity of 793,000 mPa·s at the ratio of the organopolysiloxane elastomer microparticle to the dimethyl polysiloxane being 25/75 by mass. This paste composition was designated as "Paste Composition H".

[Synthesis Example 9]

In to an emulsifying vessel were added 66.0 parts by mass of the methyl hydrogen polysiloxane shown by the average composition formula (12) and 234.0 parts by mass of the methyl vinyl polysiloxane shown by the average composition formula (13); and then, they were mixed by using a homomixer with stirring at 2000 rpm. Into the resulting mixture were added 0.32 parts by mass of polyoxyethylene lauryl ether (added mole of ethyleneoxide being 4), 0.42 parts by mass of polyoxyethylene lauryl ether (added mole of ethyleneoxide being 23), and 50 parts by mass of water; and then, they were stirred by using a homomixer at 6000 rpm for 15 minutes. Thereafter, 230 parts by mass of water was added into this mixture with stirring thereof at 2000 rpm to obtain a white and homogeneous emulsion.

Then, this emulsion was charged into a reactor equipped with an anchor agitator; and after the temperature thereof was made 15 to 20°C, the mixture solution of 0.46 parts by mass of the toluene solution containing 0.5% by mass of chloroplatinic acid, 0.85 parts by mass of polyoxyethylene lauryl ether (added mole of ethyleneoxide being 4), and 0.85 parts by mass of polyoxyethylene lauryl ether (added mole of ethyleneoxide being 23) was added into the emulsion with agitating the emulsion, and then they were agitated for 12 hours with keeping the temperature thereof at 15 to 20°C to obtain an aqueous disperse solution of organopolysiloxane elastomer microparticles. The form of this organopolysiloxane elastomer microparticle was confirmed spherical by an optical microscope; and the volume-average particle diameter thereof was 8 μm as measured by the before-mentioned Multisizer 3.

The obtained aqueous disperse solution of the organopolysiloxane elastomer microparticles was filtrated by using a filter press; and then, the solid material thus separated was transferred to a tray made of a stainless steel, dried at 105°C by using a dryer of a hot-air circulation type, and then the dried mass was crushed by a jet mill to obtain free-flow microparticles. The form of this microparticle was spherical by observation with an electron microscope; and the volume-average particle diameter thereof after dispersed into water containing a surfactant was 8 μm as measured by the before-mentioned Multisizer 3. This organopolysiloxane elastomer microparticle was designated as "Elastomer Microparticle I".

Into a glass bottle were taken 5.0 g of Elastomer Microparticle I and 50 g of the methyl polysiloxane shown in Table 1; and then, after the bottle was shaken for 30 minutes, it was allowed to stand at room temperature for 3 days. Thereafter, a solid portion was separated by a filter press, and then a mass of the solid cake was measured to obtain an oil-absorption amount thereof by the equation shown in Synthesis Example 1.

The oil-absorption amount was obtained similarly by using 50 g of the decamethyl pentasiloxane shown in Table 1 in place of 50 g of the methyl polysiloxane.

Each result thereof is shown in Table 1.

Further, 100 parts by mass of Elastomer Microparticle I and 300 parts by mass of the dimethyl polysiloxane (dynamic viscosity of 6 $mm^2$/second) were mixed and then fully kneaded by a three-roll mill to obtain a paste composition having the viscosity of 877,000 mPa·s at the ratio of the organopolysiloxane elastomer microparticle to the dimethyl polysiloxane being 25/75 by mass. This paste composition was designated as "Paste Composition I".

[Comparative Synthesis Example 1]

In to an emulsifying vessel were added 200.0 parts by mass of the methyl hydrogen polysiloxane shown by the average composition formula (4) and 96.0 parts by mass of the methyl vinyl polysiloxane shown by the average composition formula (5); and then, they were mixed by using a homomixer with stirring at 2000 rpm. Into the resulting mixture were added 0.69 parts by mass of polyoxyethylene lauryl ether (added mole of ethyleneoxide being 4), 0.87 parts by mass of polyoxyethylene lauryl ether (added mole of ethyleneoxide being 23), and 50 parts by mass of water; and then, they were stirred by using a homomixer at 6000 rpm for 15 minutes. Thereafter, 230 parts by mass of water was added into this mixture with stirring thereof at 2000 rpm to obtain a white and homogeneous emulsion.

Then, this emulsion was charged into a reactor equipped with an anchor agitator; and after the temperature thereof was made 15 to 20°C, the mixture solution of 0.46 parts by mass of the toluene solution containing 0.5% by mass of chloroplatinic acid, 0.44 parts by mass of polyoxyethylene lauryl ether (added mole of ethyleneoxide being 4), and 0.44 parts by mass of polyoxyethylene lauryl ether (added mole of ethyleneoxide being 23) was added into the emulsion with agitating the emulsion, and then they were agitated for 12 hours with keeping the temperature thereof at 15 to 20°C to obtain an aqueous disperse solution of organopolysiloxane elastomer microparticles. The form of this organopolysiloxane elastomer microparticle was confirmed spherical by an optical microscope; and the volume-average particle diameter thereof was 1.8 μm as measured by the before-mentioned Multisizer 3.

The obtained aqueous disperse solution of the organopolysiloxane elastomer microparticles was filtrated by using a filter press; and then, the solid material thus separated was transferred to a tray made of a stainless steel, dried at 105°C by using a dryer of a hot-air circulation type, and then the dried mass was crushed by a jet mill to obtain free-flow microparticles. The form of this microparticle was spherical by observation with an electron microscope; and the volume-average particle diameter thereof after dispersed into water containing a surfactant was 1.8 μm as measured by the before-mentioned Multisizer 3. This organopolysiloxane elastomer microparticle was designated as "Elastomer Microparticle J".

Into a glass bottle were taken 5.0 g of Elastomer Microparticle J and 50 g of the methyl polysiloxane shown in Table 1; and then, after the bottle was shaken for 30 minutes, it was allowed to stand at room temperature for 3 days. Thereafter, a solid portion was separated by a filter press, and then a mass of the solid cake was measured to obtain an oil-absorption amount thereof by the equation shown in Synthesis Example 1.

The oil-absorption amount was obtained similarly by using 50 g of the decamethyl pentasiloxane shown in Table 1 in place of 50 g of the methyl polysiloxane.

Each result thereof is shown in Table 1.

Further, 100 parts by mass of Elastomer Microparticle J and 335 parts by mass of the dimethyl polysiloxane (dynamic viscosity of 6 $mm^2$/second) were mixed and then fully kneaded by a three-roll mill to obtain a paste composition having the viscosity of 894,000 mPa·s at the ratio of the organopolysiloxane elastomer microparticle to the dimethyl polysiloxane being 23/77 by mass. This paste composition was designated as "Paste Composition J".

[Comparative Synthesis Example 2]

In to an emulsifying vessel were added 105.0 parts by mass of the methyl hydrogen polysiloxane shown by the average composition formula (8) and 195.0 parts by mass of the methyl vinyl polysiloxane shown by the average composition formula (9); and then, they were mixed by using a homomixer with stirring at 2000 rpm. Into the resulting mixture were added 0.48 parts by mass of polyoxyethylene lauryl ether (added mole of ethyleneoxide being 4), 0.36 parts by mass of polyoxyethylene lauryl ether (added mole of ethyleneoxide being 23), and 100 parts by mass of water; and then, they were stirred by using a homomixer at 4500 rpm for 15 minutes. Thereafter, 180 parts by mass of water was added into this mixture with stirring thereof at 2000 rpm to obtain a white and homogeneous emulsion.

Then, this emulsion was charged into a reactor equipped with an anchor agitator; and after the temperature thereof was made 15 to 20°C, the mixture solution of 0.46 parts by mass of the toluene solution containing 0.5% by mass of chloroplatinic acid, 0.80 parts by mass of polyoxyethylene lauryl ether (added mole of ethyleneoxide being 4), and 0.80 parts by mass of polyoxyethylene lauryl ether (added mole of ethyleneoxide being 23) was added into the emulsion with agitating the emulsion, and then they were agitated for 12 hours with keeping the temperature thereof at 15 to 20°C to obtain an aqueous disperse solution of organopolysiloxane elastomer microparticles. The form of this organopolysiloxane elastomer microparticle was confirmed spherical by an optical microscope; and the volume-average particle diameter thereof was 52 μm as measured by the before-mentioned Multisizer 3.

The obtained aqueous disperse solution of the organopolysiloxane elastomer microparticles was filtrated by using a filter press; and then, the solid material thus separated was transferred to a tray made of a stainless steel, dried at 105°C by using a dryer of a hot-air circulation type, and then the dried mass was crushed by a jet mill to obtain free-flow microparticles. The form of this microparticle was spherical by observation with an electron microscope; and the volume-average particle diameter thereof after dispersed into water containing a surfactant was 52 μm as measured by the before-mentioned Multisizer 3. This organopolysiloxane elastomer microparticle was designated as "Elastomer Microparticle K".

Into a glass bottle were taken 5.0 g of Elastomer Microparticle K and 50 g of the methyl polysiloxane shown in Table 1; and then, after the bottle was shaken for 30 minutes, it was allowed to stand at room temperature for 3 days. Thereafter, a solid portion was separated by a filter press, and then a mass of the solid cake was measured to obtain an oil-absorption amount thereof by the equation shown in Synthesis Example 1.

The oil-absorption amount was obtained similarly by using 50 g of the decamethyl pentasiloxane shown in Table 1 in place of 50 g of the methyl polysiloxane.

Each result thereof is shown in Table 1.

Further, 100 parts by mass of Elastomer Microparticle K and 285 parts by mass of the dimethyl polysiloxane (dynamic viscosity of 6 $mm^2$/second) were mixed and then fully kneaded by a three-roll mill to obtain a paste composition having the viscosity of 659,000 mPa·s at the ratio of the organopolysiloxane elastomer microparticle to the dimethyl polysiloxane being 26/74 by mass. This paste composition was designated as "Paste Composition K".

[Comparative Synthesis Example 3]

In to an emulsifying vessel were added 11.0 parts by mass of the methyl hydrogen polysiloxane shown by the following average composition formula (14) and 289.0 parts by mass of the methyl vinyl polysiloxane shown by the following average composition formula (15); and then, they were mixed by using a homomixer with stirring at 2000 rpm. Into the resulting mixture were added 0.7 parts by mass of polyoxyethylene lauryl ether (added mole of ethyleneoxide being 9) and 60 parts by mass of water; and then, they were stirred by using a homomixer at 6000 rpm for 15 minutes. Thereafter, 220 parts by mass of water was added into this mixture with stirring thereof at 2000 rpm to obtain a white and homogeneous emulsion.

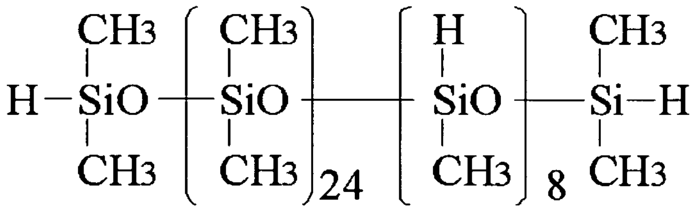
(14)

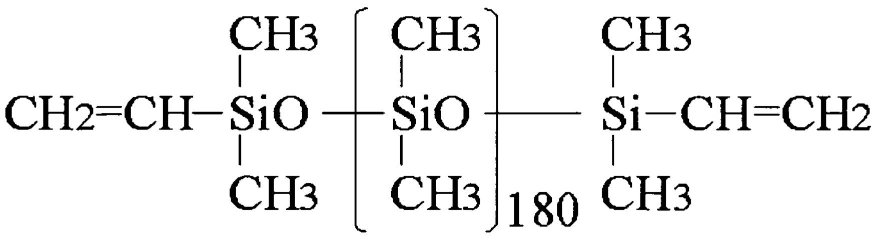
(15)

Then, this emulsion was charged into a reactor equipped with an anchor agitator; and after the temperature thereof was made 15 to 20°C, the mixture solution of 0.46 parts by mass of the toluene solution containing 0.5% by mass of chloroplatinic acid, 0.87 parts by mass of polyoxyethylene lauryl ether (added mole of ethyleneoxide being 4), and 0.87 parts by mass of polyoxyethylene lauryl ether (added mole of ethyleneoxide being 23) was added into the emulsion with agitating the emulsion, and then they were agitated for 12 hours with keeping the temperature thereof at 15 to 20°C to obtain an aqueous disperse solution of organopolysiloxane elastomer microparticles. The form of this organopolysiloxane elastomer microparticle was confirmed spherical by an optical microscope; and the volume-average particle diameter thereof was 12 μm as measured by the before-mentioned Multisizer 3.

The obtained aqueous disperse solution of the organopolysiloxane elastomer microparticles was filtrated by using a filter press; and then, the solid material thus separated was transferred to a tray made of a stainless steel, dried at 105°C by using a dryer of a hot-air circulation type, and then the dried mass was crushed by a jet mill to obtain free-flow microparticles. The form of this microparticle was spherical by observation with an electron microscope; and the volume-average particle diameter thereof after dispersed into water containing a surfactant was 12 μm as measured by the before-mentioned Multisizer 3. This organopolysiloxane elastomer microparticle was designated as "Elastomer Microparticle L".

Into a glass bottle were taken 5.0 g of Elastomer Microparticle L and 50 g of the methyl polysiloxane shown in Table 1; and then, after the bottle was shaken for 30 minutes, it was allowed to stand at room temperature for 3 days. Thereafter, a solid portion was separated by a filter press, and then a mass of the solid cake was measured to obtain an oil-absorption amount thereof by the equation shown in Synthesis Example 1.

The oil-absorption amount was obtained similarly by using 50 g of the decamethyl pentasiloxane shown in Table 1 in place of 50 g of the methyl polysiloxane.

Each result thereof is shown in Table 1.

Further, 100 parts by mass of Elastomer Microparticle L and 300 parts by mass of the dimethyl polysiloxane (dynamic viscosity of 6 $mm^2$/second) were mixed and then fully kneaded by a three-roll mill to obtain a paste composition having the viscosity of 778,000 mPa·s at the ratio of the organopolysiloxane elastomer microparticle to the dimethyl polysiloxane being 25/75 by mass. This paste composition was designated as "Paste Composition L".

[Comparative Synthesis Example 4]

In to an emulsifying vessel were added 6.0 parts by mass of the methyl hydrogen polysiloxane shown by the following average composition formula (16) and 294.0 parts by mass of the methyl vinyl polysiloxane shown by the average composition formula (15); and then, they were mixed by using a homomixer with stirring at 2000 rpm. Into the resulting mixture were added 0.7 parts by mass of polyoxyethylene lauryl ether (added mole of ethyleneoxide being 9) and 60 parts by mass of water; and then, they were stirred by using a homomixer at 6000 rpm for 15 minutes. Thereafter, 220 parts by mass of water was added into this mixture with stirring thereof at 2000 rpm to obtain a white and homogeneous emulsion.

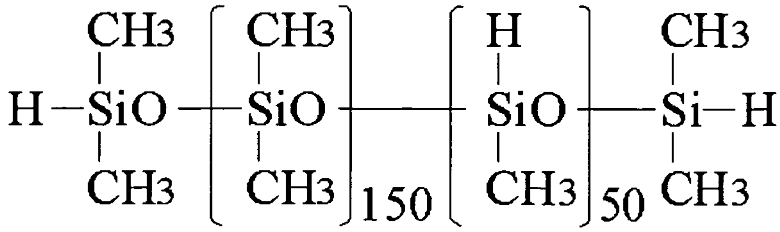

(16)

Then, this emulsion was charged into a reactor equipped with an anchor agitator; and after the temperature thereof was made 15 to 20°C, the mixture solution of 0.46 parts by mass of the toluene solution containing 0.5% by mass of chloroplatinic acid, 0.87 parts by mass of polyoxyethylene lauryl ether (added mole of ethyleneoxide being 4), and 0.87 parts by mass of polyoxyethylene lauryl ether (added mole of ethyleneoxide being 23) was added into the emulsion with agitating the emulsion, and then they were agitated for 12 hours with keeping the temperature thereof at 15 to 20°C to obtain an aqueous disperse solution of organopolysiloxane elastomer microparticles. The form of this organopolysiloxane elastomer microparticle was confirmed spherical by an optical microscope; and the volume-average particle diameter thereof was 12 μm as measured by the before-mentioned Multisizer 3.

The obtained aqueous disperse solution of the organopolysiloxane elastomer microparticles was filtrated by using a filter press; and then, the solid material thus separated was transferred to a tray made of a stainless steel, dried at 105°C by using a dryer of a hot-air circulation type, and then the dried mass was crushed by a jet mill to obtain free-flow microparticles. The form of this microparticle was spherical by observation with an electron microscope; and the volume-average particle diameter thereof after dispersed into water containing a surfactant was 12 μm as measured by the before-mentioned Multisizer 3. This organopolysiloxane elastomer microparticle was designated as "Elastomer Microparticle M".

Into a glass bottle were taken 5.0 g of Elastomer Microparticle M and 50 g of the methyl polysiloxane shown in Table 1; and then, after the bottle was shaken for 30 minutes, it was allowed to stand at room temperature for 3 days. Thereafter, a solid portion was separated by a filter press, and then a mass of the solid cake was measured to obtain an oil-absorption amount thereof by the equation shown in Synthesis Example 1.

The oil-absorption amount was obtained similarly by using 50 g of the decamethyl pentasiloxane shown in Table 1 in place of 50 g of the methyl polysiloxane.

Each result thereof is shown in Table 1.

Further, 100 parts by mass of Elastomer Microparticle M and 203 parts by mass of the dimethyl polysiloxane (dynamic viscosity of 6 $mm^2$/second) were mixed and then fully kneaded by a three-roll mill to obtain a paste composition having the viscosity of 814,000 mPa·s at the ratio of the organopolysiloxane elastomer microparticle to the dimethyl polysiloxane being 33/67 by mass. This paste composition was designated as "Paste Composition M".

[Comparative Synthesis Example 5]

In to an emulsifying vessel were added 5.0 parts by mass of the methyl hydrogen polysiloxane shown by the average composition formula (14) and 295.0 parts by mass of the methyl vinyl polysiloxane shown by the following average composition formula (17); and then, they were mixed by using a homomixer with stirring at 2000 rpm. Into the resulting mixture were added 0.7 parts by mass of polyoxyethylene lauryl ether (added mole of ethyleneoxide being 9) and 60 parts by mass of water; and then, they were stirred by using a homomixer at 6000 rpm for 15 minutes. Thereafter, 220 parts by mass of water was added into this mixture with stirring thereof at 2000 rpm to obtain a white and homogeneous emulsion.

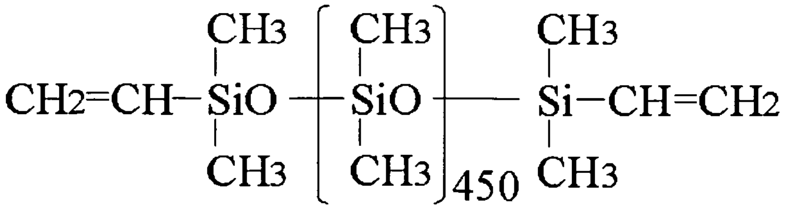

(17)

Then, this emulsion was charged into a reactor equipped with an anchor agitator; and after the temperature thereof was made 15 to 20°C, the mixture solution of 0.46 parts by mass of the toluene solution containing 0.5% by mass of chloroplatinic acid, 0.87 parts by mass of polyoxyethylene lauryl ether (added mole of ethyleneoxide being 4), and 0.87 parts by mass of polyoxyethylene lauryl ether (added mole of ethyleneoxide being 23) was added into the emulsion with agitating the emulsion, and then they were agitated for 12 hours with keeping the temperature thereof at 15 to 20°C to obtain an aqueous disperse solution of organopolysiloxane elastomer microparticles. The form of this organopolysiloxane elastomer microparticle was confirmed spherical by an optical microscope; and the volume-average particle diameter thereof was 12 μm as measured by the before-mentioned Multisizer 3.

The obtained aqueous disperse solution of the organopolysiloxane elastomer microparticles was filtrated by using a filter press; and then, the solid material thus separated was transferred to a tray made of a stainless steel, dried at 105°C by using a dryer of a hot-air circulation type, and then the dried mass was crushed by a jet mill to obtain free-flow microparticles. The form of this microparticle was spherical by observation with an electron microscope; and the volume-average particle diameter thereof after dispersed into water containing a surfactant was 12 μm as measured by the before-mentioned Multisizer 3. This organopolysiloxane elastomer microparticle was designated as "Elastomer Microparticle N".

Into a glass bottle were taken 5.0 g of Elastomer Microparticle N and 50 g of the methyl polysiloxane shown in Table 1; and then, after the bottle was shaken for 30 minutes, it was allowed to stand at room temperature for 3 days. Thereafter, a solid portion was separated by a filter press, and then a mass of the solid cake was measured to obtain an oil-absorption amount thereof by the equation shown in Synthesis Example 1.

The oil-absorption amount was obtained similarly by using 50 g of the decamethyl pentasiloxane shown in Table 1 in place of 50 g of the methyl polysiloxane.

Each result thereof is shown in Table 1.

Further, 100 parts by mass of Elastomer Microparticle N and 257 parts by mass of the dimethyl polysiloxane (dynamic viscosity of 6 $mm^2$/second) were mixed and then fully kneaded by a three-roll mill to obtain a paste composition having the viscosity of 724,000 mPa·s at the ratio of the organopolysiloxane elastomer microparticle to the dimethyl polysiloxane being 28/72 by mass. This paste composition was designated as "Paste Composition N".

[Comparative Synthesis Example 6]

In to an emulsifying vessel were added 190.0 parts by mass of the methyl hydrogen polysiloxane shown by the average composition formula (6) and 110.0 parts by mass of the methyl vinyl polysiloxane shown by the following average composition formula (18); and then, they were mixed by using a homomixer with stirring at 2000 rpm. Into the resulting mixture were added 0.7 parts by mass of polyoxyethylene lauryl ether (added mole of ethyleneoxide being 9) and 60 parts by mass of water; and then, they were stirred by using a homomixer at 6000 rpm for 15 minutes. Thereafter, 220 parts by mass of water was added into this mixture with stirring thereof at 2000 rpm to obtain a white and homogeneous emulsion.

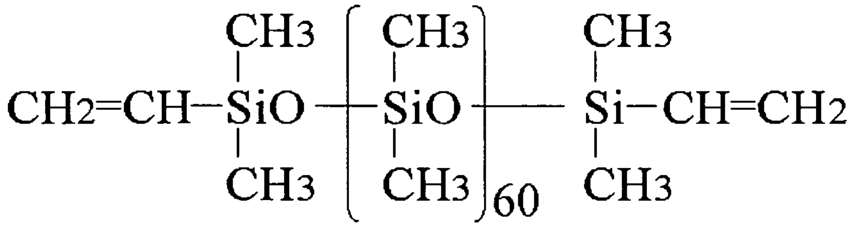

(18)

Then, this emulsion was charged into a reactor equipped with an anchor agitator; and after the temperature thereof was made 15 to 20°C, the mixture solution of 0.46 parts by mass of the toluene solution containing 0.5% by mass of chloroplatinic acid, 0.87 parts by mass of polyoxyethylene lauryl ether (added mole of ethyleneoxide being 4), and 0.87 parts by mass of polyoxyethylene lauryl ether (added mole of ethyleneoxide being 23) was added into the

emulsion with agitating the emulsion, and then they were agitated for 12 hours with keeping the temperature thereof at 15 to 20°C to obtain an aqueous disperse solution of organopolysiloxane elastomer microparticles. The form of this organopolysiloxane elastomer microparticle was confirmed spherical by an optical microscope; and the volume-average particle diameter thereof was 11 μm as measured by the before-mentioned Multisizer 3.

The obtained aqueous disperse solution of the organopolysiloxane elastomer microparticles was filtrated by using a filter press; and then, the solid material thus separated was transferred to a tray made of a stainless steel, dried at 105°C by using a dryer of a hot-air circulation type, and then the dried mass was crushed by a jet mill to obtain free-flow microparticles. The form of this microparticle was spherical by observation with an electron microscope; and the volume-average particle diameter thereof after dispersed into water containing a surfactant was 11 μm as measured by the before-mentioned Multisizer 3. This organopolysiloxane elastomer microparticle was designated as "Elastomer Microparticle O".

Into a glass bottle were taken 5.0 g of Elastomer Microparticle O and 50 g of the methyl polysiloxane shown in Table 1; and then, after the bottle was shaken for 30 minutes, it was allowed to stand at room temperature for 3 days. Thereafter, a solid portion was separated by a filter press, and then a mass of the solid cake was measured to obtain an oil-absorption amount thereof by the equation shown in Synthesis Example 1.

The oil-absorption amount was obtained similarly by using 50 g of the decamethyl pentasiloxane shown in Table 1 in place of 50 g of the methyl polysiloxane.

Each result thereof is shown in Table 1.

Further, 100 parts by mass of Elastomer Microparticle O and 257 parts by mass of the dimethyl polysiloxane (dynamic viscosity of 6 $mm^2$/second) were mixed and then fully kneaded by a three-roll mill to obtain a paste composition having the viscosity of 737,000 mPa·s at the ratio of the organopolysiloxane elastomer microparticle to the dimethyl polysiloxane being 28/72 by mass. This paste composition was designated as "Paste Composition O".

[Table 1]

| | Elastomer Microparticle | Oil-absorption amount (g/5-g of elastomer microparticle) | |
|---|---|---|---|
| | | Decamethyl pentasiloxane (dynamic viscosity of 4.0 $mm^2$/second) | Dimethyl polysiloxane (dynamic viscosity of 6.0 $mm^2$/second) |
| Synthesis Example 1 | A | 26 | 20 |
| Synthesis Example 2 | B | 27 | 20 |
| Synthesis Example 3 | C | 26 | 20 |
| Synthesis Example 4 | D | 24 | 18 |
| Synthesis Example 5 | E | 27 | 20 |
| Synthesis Example 6 | F | 26 | 20 |
| Synthesis Example 7 | G | 29 | 22 |
| Synthesis Example 8 | H | 28 | 21 |
| Synthesis Example 9 | I | 28 | 21 |
| Comparative Synthesis Example 1 | J | 29 | 22 |
| Comparative Synthesis Example 2 | K | 24 | 18 |
| Comparative Synthesis Example 3 | L | 16 | 10 |
| Comparative Synthesis Example 4 | M | 15 | 9 |
| Comparative Synthesis Example 5 | N | 17 | 12 |
| Comparative Synthesis Example 6 | O | 20 | 13 |

[Examples 1 to 9 and Comparative Examples 1 to 6]

Each of the paste compositions A to O obtained in Synthesis Examples 1 to 9 and Comparative Synthesis Examples 1 to 6 was applied to skins of 10 expert panelists and evaluated as to the feeling thereof according to the criteria shown in Table 2. Judgment was made according to the criteria shown in Table 3 based on the average value obtained. Judgment results thereof are shown in Table 4.

Further, the frictional resistance force of each of the paste compositions A to O obtained in Synthesis Examples 1 to 9 and Comparative Synthesis Examples 1 to 6 was measured by using a tactile meter (Portable tactile meter TYPE:33 (manufactured by SHINTO scientific Co. Ltd.)), and thereby "slipperiness" (the frictional resistance force of the oil which was used for the paste composition / the frictional resistance force of the paste composition) was obtained. The tactile meter was a frictional resistance force-measuring apparatus which was capable of, at the time of frictioning friction surface by a finger, measuring a vertical-load of the finger and a frictional resistance force which the finger feels at the same time, and an artificial leather (Artificial Leather SUPPLALE (made by IDEMITSU TECHNOFINE Co. Ltd.)) was set as the friction surface. Each of the paste compositions (5 $mg/cm^2$) was applied onto the friction surface (the artificial leather), and then twenty-reciprocatory actions were conducted with a finger at the speed of two seconds per one-reciprocatory action. This measurement was conducted 3 times and the average of the frictional resistance force was obtained. Evaluation was conducted based on "slipperiness", that is "the frictional resistance force of the oil which is used for the paste composition / the frictional resistance force of the paste composition". The closer this "slipperiness" is to "1", or the bigger this "slipperiness" is than "1", the less the composition impairs the feel of the oil and the more sufficiently the composition keeps paste-form, that is, the more excellent smoothness, lightness, and spread-ability the composition has. The measurement results were shown in Table 5.

[Table 2]

| Score | Items | | | |
|---|---|---|---|---|
| | Sleek feeling | Oil-film feeling | Light feeling | Spreading |
| 5 | Strongly felt | None | Light | Good |
| 4 | Felt | Very little | Comparatively light | Comparatively good |
| 3 | Somewhat felt | Little | Fair | Fair |
| 2 | Slightly felt | Some | Comparatively heavy | Comparatively bad |
| 1 | Not felt | Strong | Heavy | Bad |

[Table 3]

| Criteria | Judgment |
|---|---|
| Obtained average score of 4.5 or more | Excellent |
| Obtained average score of 3.5 or more and less than 4.5 | Good |
| Obtained average score of 2.5 or more and less than 3.5 | Fair |
| Obtained average score of 1.5 or more and less than 2.5 | Poor |
| Obtained average score of less than 1.5 | Bad |

[Table 4]

| | | Paste Composition | Items | | | |
|---|---|---|---|---|---|---|
| | | | Powdery feeling | Oil-film feeling | Light feeling | Spreading |
| | 1 | A | Excellent | Excellent | Excellent | Excellent |
| | 2 | B | Excellent | Excellent | Excellent | Excellent |
| | 3 | C | Excellent | Excellent | Excellent | Excellent |
| | 4 | D | Excellent | Excellent | Excellent | Excellent |

(continued)

| | | Paste Composition | Items | | | |
|---|---|---|---|---|---|---|
| | | | Powdery feeling | Oil-film feeling | Light feeling | Spreading |
| Examples | 5 | E | Excellent | Excellent | Excellent | Excellent |
| | 6 | F | Excellent | Excellent | Excellent | Excellent |
| | 7 | G | Excellent | Excellent | Excellent | Excellent |
| | 8 | H | Excellent | Excellent | Excellent | Excellent |
| | 9 | I | Excellent | Excellent | Excellent | Excellent |
| Comparative Examples | 1 | J | Excellent | Excellent | Fair | Fair |
| | 2 | K | Fair | Fair | Fair | Good |
| | 3 | L | Fair | Fair | Good | Good |
| | 4 | M | Poor | Poor | Fair | Good |
| | 5 | N | Fair | Fair | Fair | Excellent |
| | 6 | O | Good | Fair | Fair | Excellent |

[Table 5]

| | | Paste Composition | Slipperiness |
|---|---|---|---|
| Examples | 1 | A | 1.06 |
| | 2 | B | 0.90 |
| | 3 | C | 1.11 |
| | 4 | D | 1.06 |
| | 5 | E | 1.18 |
| | 6 | F | 1.14 |
| | 7 | G | 0.90 |
| | 8 | H | 1.03 |
| | 9 | I | 0.94 |
| Comparative Examples | 1 | J | 0.59 |
| | 2 | K | 0.61 |
| | 3 | L | 0.71 |
| | 4 | M | 0.57 |
| | 5 | N | 0.60 |
| | 6 | O | 0.68 |

As shown in Table 4, the paste organopolysiloxane elastomer compositions of the present invention in Examples 1 to 9 (Paste compositions A to I) gave a light powdery feeling without an oil-film feeling as compared with the paste compositions of Comparative Synthesis Examples 1 to 6 (Paste Compositions J to O).

As shown in Table 5, the paste organopolysiloxane elastomer compositions of the present invention in Examples 1 to 9 (Paste compositions A to I) gave more excellent slipperiness than the paste compositions of Comparative Synthesis Examples 1 to 6 (Paste Compositions J to O).

Then, Example of each cosmetic containing the organopolysiloxane elastomer composition of the present invention will be shown.

[Example 10: w/o milky lotion]

| (Components) | (parts by mass) |
|---|---|
| 1. Paste Composition A obtained in Synthesis Example1 | 15.0 |
| 2. Dimethyl polysiloxane (viscosity: 6 $mm^2$/second) | 12.0 |
| 3. Decamethyl cyclopentasiloxane | 10.0 |
| 4. Glyceryl trioctanoate | 5.0 |
| 5. Polyether-modified silicone (note 1) | 3.0 |
| 6. 1,3-butanediol | 5.0 |
| 7. Preservative | appropriate amount |
| 8. Fragrance | appropriate amount |
| 9. Purified water | 50.0 |

(note 1: KF-6017, manufactured by Shin-Etsu Chemical Co., Ltd.)

(Preparation method)

a: Components 1 to 5 were uniformly mixed by agitation.

b: Components 6 to 9 were uniformly mixed, and then added into "a"; and then, they were mixed by agitation to obtain an emulsion.

(Result)

The emulsion thus obtained gave a w/o milky lotion finished with a shiny look, with a wide and light spreading and a refreshing feeling, and without stickiness.

[Example 11: o/w cream]

| (Components) | (parts by mass) |
|---|---|
| 1. Paste Composition B obtained in Synthesis Example 2 | 8.0 |
| 2. Crosslinking methyl phenyl polysiloxane (note 2) | 2.0 |
| 3. Isotridecyl isononanoate | 5.0 |
| 4. Dipropylene glycol | 7.0 |
| 5. Glycerin | 5.0 |
| 6. 2% Methyl cellulose solution (note 3) | 7.0 |
| 7. Polyacrylamide emulsifier (note 4) | 2.0 |
| 8. Guanine | 1.0 |
| 9. Preservative | appropriate amount |
| 10. Fragrance | appropriate amount |
| 11. Purified water | 63.0 |

(note 2: KSG-18, manufactured by Shin-Etsu Chemical Co., Ltd.)
(note 3: Metolose SM-4000, manufactured by Shin-Etsu Chemical Co., Ltd.)
(note 4: Sepigel 305, manufactured by SEPIC S. A.)

(Preparation method)

a: Components 3 to 11 were uniformly mixed by agitation.

b: Components 1 to 2 were uniformly mixed, and then added into "a"; and then, they were mixed by agitation to obtain an emulsion (cream).

(Result)

The cream thus obtained gave a fine o/w cream with a wide and light spreading and a refreshing use feeling without stickiness and greasiness, and in addition, with good durability and stability.

[Example 12: w/o cream]

| (Components) | (parts by mass) |
|---|---|
| 1. Paste Composition D obtained in Synthesis Example 4 | 5.0 |
| 2. Dimethyl polysiloxane (viscosity: 6 $mm^2$/second) | 10.0 |
| 3. Crosslinking polyether-modified silicone (note 5) | 2.0 |
| 4. Polyether-modified branched silicone (note 6) | 0.5 |
| 5. Dipropylene glycol | 10.0 |
| 6. Sodium citrate | 0.2 |
| 7. Ethanol | 5.0 |
| 8. Preservative | appropriate amount |
| 9. Fragrance | appropriate amount |
| 10. Purified water | 67.3 |

(note 5: KSG-210, manufactured by Shin-Etsu Chemical Co., Ltd.)
(note 6: KF-6028, manufactured by Shin-Etsu Chemical Co., Ltd.)

(Preparation method)

a: Components 1 to 4 were uniformly mixed by agitation.
b: Components 5 to 10 were uniformly mixed, and then added into "a"; and then, they were mixed by agitation to obtain an emulsion.

(Result)

The cream thus obtained gave a w/o cream with a wide and light spreading and a vivid, refreshing use feeling without greasiness and stickiness.

[Example 13: powder foundation]

| (Components) | (parts by mass) |
|---|---|
| 1. Paste Composition E obtained in Synthesis Example 5 | 6.0 |
| 2. Acrylate/dimethyl silicone copolymer (note 7) | 3.0 |
| 3. Squalane | 3.0 |
| 4. Mica treated with acryl silicone (note 8) | 50.0 |
| 5. Talc treated with triethoxy caprylyl (note 9) | 24.0 |
| 6. Titanium oxide treated with triethoxy alkly silicone (note 10) | 9.0 |
| 7. Hybrid silicone composite powder (note 11) | 2.0 |
| 8. Spherical polymethyl silsesquioxane powder (note 12) | 3.0 |
| 9. Pigment treated with triethoxy alkyl silicone (note 13) | appropriate amount |
| 10. Preservative | appropriate amount |

(continued)

| (Components) | (parts by mass) |
|---|---|
| 11. Fragrance | appropriate amount |

(note 7: KP-561P, manufactured by Shin-Etsu Chemical Co., Ltd.)
(note 8: Mica treated with KP-574, manufactured by Shin-Etsu Chemical Co., Ltd.)
(note 9: Talc treated with AES-3083, manufactured by Shin-Etsu Chemical Co., Ltd.)
(note 10: Titanium oxide treated with KF-9909, manufactured by Shin-Etsu Chemical Co., Ltd.)
(note 11: KSP-300, manufactured by Shin-Etsu Chemical Co., Ltd.)
(note 12: KMP-590, manufactured by Shin-Etsu Chemical Co., Ltd.)
(note 13: Pigment treated with KF-9909, manufactured by Shin-Etsu Chemical Co., Ltd.)

(Preparation method)

a: Components 4 to 10 were uniformly mixed.
b: Components 1 to 3 were uniformly mixed, and then added into "a"; and then, they were mixed.
c: After component 11 was added thereinto, the resulting mixture was press molded in a mold to obtain a powder foundation.

(Result)

The powder foundation thus obtained showed excellent adhesion with a light and wide spreading feeling without stickiness.

[Example 14: w/o cream foundation]

| (Components) | (parts by mass) |
|---|---|
| 1. Paste Composition H obtained in Synthesis Example 8 | 8.0 |
| 2. Crosslinking polyether-modified silicone (note 14) | 3.0 |
| 3. Polyether-modified silicone (note 15) | 1.0 |
| 4. Dimethyl polysiloxane (viscosity: 6 $mm^2$/second) | 3.0 |
| 5. Decamethyl pentasiloxane | 9.0 |
| 6. Glyceryl trioctanoate | 5.0 |
| 7. Neopentyl glycol dioctanoate | 2.0 |
| 8. Spherical polymethyl silsesquioxane powder (note 16) | 1.5 |
| 9. Branched silicone co-modified with polyglycerin and alkyl (note 17) | 2.0 |
| 10. Pigment treated with triethoxy alkyl silicone (note 18) | 5.0 |
| 11. Pentylene glycol | 5.0 |
| 12. Sodium chloride | 0.5 |
| 13. Preservative | appropriate amount |
| 14. Fragrance | appropriate amount |
| 15. Purified water | 55.0 |

(note 14: KSG-210, manufactured by Shin-Etsu Chemical Co., Ltd.)
(note 15: KF-6017, manufactured by Shin-Etsu Chemical Co., Ltd.)
(note 16: KMP-590, manufactured by Shin-Etsu Chemical Co., Ltd.)
(note 17: KF-6105, manufactured by Shin-Etsu Chemical Co., Ltd.)
(note 18: Pigment treated with KF-9909, manufactured by Shin-Etsu Chemical Co., Ltd.)

(Preparation method)

a: Components 1 to 4, a part of component 5, and components 6 to 8 were uniformly mixed by agitation.
b: Components 9 to 10 and the rest of component 5 were uniformly mixed by agitation.
c: Components 11 to 13 and 15 were uniformly mixed.
d: "c" was added into "a", and then they were mixed by agitation for emulsification.
e: Component 14 and "b" were added into "d", and then they were mixed by agitation.

(Result)

The cream foundation thus obtained gave a w/o cream foundation with a light and wide spreading feeling, good pigment dispersion, and excellent adhesion, and without greasiness and stickiness.

[Example 15: eye shadow]

| (Components) | (parts by mass) |
|---|---|
| 1. Sericite | 45.0 |
| 2. Mica | 12.0 |
| 3. Talc | 12.0 |
| 4. Titanium oxide | 10.0 |
| 5. Titanium oxide microparticle | 5.0 |
| 6. Magnesium stearate | 3.0 |
| 7. Pigment | appropriate amount |
| 8. Octyl dodecanol | 3.0 |
| 9. Dimethyl polysiloxane (viscosity: 6 $mm^2$/second) | 4.0 |
| 10. Paste Composition E obtained in Synthesis Example 5 | 6.0 |
| 11. Preservative | appropriate amount |
| 12. Fragrance | appropriate amount |

(Preparation method)

a: Components 8 to 11 were uniformly mixed.
b: Components 1 to 7 were uniformly mixed, and then added into "a"; and then, they were mixed.
c: Component 12 was added into "b".

(Result)

The eye shadow thus obtained was excellent in spreading, adhesion, and cosmetic durability.

[Example 16: cream eye color]

| (Components) | (parts by mass) |
|---|---|
| 1. Paste Composition E obtained in Synthesis Example 5 | 3.5 |
| 2. Paste Composition G obtained in Synthesis Example 7 | 5.0 |
| 3. Polyether-modified branched siloxane (note 19) | 2.0 |
| 4. Dimethyl polysiloxane (viscosity: 6 $mm^2$/second) | 6.5 |
| 5. Decamethyl pentasiloxane | 26.6 |
| 6. Triethyl hexanoin | 5.0 |
| 7. Organic-modified bentonite | 1.2 |
| 8. Acrylate/dimethyl silicone copolymer (note 20) | 1.5 |
| 9. Pigment treated with triethoxy alkyl silicone (note 21) | 5.0 |

(continued)

| (Components) | (parts by mass) |
|---|---|
| 10. Dipropylene glycol | 5.0 |
| 11. Sodium citrate | 0.2 |
| 12. Preservative | appropriate amount |
| 13. Fragrance | appropriate amount |
| 14. Purified water | 38.5 |

(note 19: KF-6028, manufactured by Shin-Etsu Chemical Co., Ltd.)
(note 20: KP-575, manufactured by Shin-Etsu Chemical Co., Ltd.)
(note 21: Pigment treated with KF-9909, manufactured by Shin-Etsu Chemical Co., Ltd.)

(Preparation method)

a: Components 1 to 4, a part of component 5, and components 6 to 7 were uniformly mixed by agitation.
b: Components 10 to 12 and component 14 were mixed by agitation.
c: Components 8 to 9 and the rest of component 5 were uniformly mixed.
d: "c" was added into "a", and then they were mixed by agitation for emulsification.
e: Component 13 and "b" were added into "d", and then they were mixed by agitation.

(Result)

The cream eye color thus obtained was excellent in adhesion and pigment dispersion with a light and wide spreading feeling and without greasiness and stickiness.

[Example 17: roll-on type antiperspirant]

| (Components) | (parts by mass) |
|---|---|
| 1. Paste Composition A obtained in Synthesis Example | 120.0 |
| 2. Crosslinking polyether-modified silicone (note 22) | 20.0 |
| 3. Crosslinking dimethyl polysiloxane (note 23) | 15.0 |
| 4. Dimethyl polysiloxane (viscosity: 6 $mm^2$/second) | 10.0 |
| 5. Decamethyl pentasiloxane | 15.0 |
| 6. Aluminum zirconium tetrachlorohydrate | 20.0 |
| 7. Fragrance | appropriate amount |

(note 22: KSG-240, manufactured by Shin-Etsu Chemical Co., Ltd.)
(note 23: KSG-15, manufactured by Shin-Etsu Chemical Co., Ltd.)

(Preparation method)

a: Components 1 to 5 were uniformly mixed.
b: Components 6 to 7 were added into "a"; and then, they were mixed for dispersion.

(Result)

The roll-on type antiperspirant thus obtained had a light and wide spreading property with a clean and cool feeling and without stickiness.

[Example 18: sun-cut cream]

| (Components) | (parts by mass) |
|---|---|
| 1. Zinc oxide treated with triethoxy alkyl silicone (note 24) | 20.0 |
| 2. Silicone co-modified with alkyl and polyglycerin (note 25) | 12.0 |
| 3. Decamethyl cyclopentasiloxane | 20.0 |
| 4. Neopentyl glycol dioctanoate | 7.0 |
| 5. Crosslinking silicone co-modified with alkyl and polyether (note 26) | 2.0 |
| 6. Paste Composition E obtained in Synthesis Example 5 | 5.0 |
| 7. Branched silicone co-modified with alkyl and polyether (note 27) | 1.0 |
| 8. Octyl methoxycinnamate | 3.0 |
| 9. Sodium citrate | 0.2 |
| 10. Dipropylene glycol | 3.0 |
| 11. Preservative | appropriate amount |
| 12. Fragrance | appropriate amount |
| 13. Purified water | 26.8 |

(note 24: Zinc oxide treated with KF-9909, manufactured by Shin-Etsu Chemical Co., Ltd.)
(note 25: KF-6105, manufactured by Shin-Etsu Chemical Co., Ltd.)
(note 26: KSG-310, manufactured by Shin-Etsu Chemical Co., Ltd.)
(note 27: KF-6038, manufactured by Shin-Etsu Chemical Co., Ltd.)

(Preparation method)

a: Components 4 to 8 and a part of component 3 were uniformly mixed by agitation.
b: Components 9 to 11 and component 13 were mixed.
c: Components 1 to 2 and the rest of component 3 were mixed.
d: "b" was added into "a", and then they were mixed by agitation for emulsification.
e: Component 12 and "c" were added into "d", and then they were mixed by agitation.

(Result)

The sun-cut cream thus obtained was excellent in adhesion and cosmetic durability with a light and wide spreading property and without greasiness and stickiness.

[Example 19: sun-cut milky lotion]

| (Components) | (parts by mass) |
|---|---|
| 1. Paste Composition G obtained in Synthesis Example 7 | 5.0 |
| 2. Paste Composition E obtained in Synthesis Example 5 | 1.0 |
| 3. Dimethyl polysiloxane (viscosity: 6 $mm^2$/second) | 5.0 |
| 4. Glyceryl trioctanoate | 2.0 |
| 5. Polyether-modified silicone (note 28) | 1.0 |
| 6. Disperse of titanium oxide in decamethyl cyclopentasiloxane (note 29) | 30.0 |
| 7. Disperse of zinc oxide in decamethyl cyclopentasiloxane (note 30) | 30.0 |
| 8. Dipropylene glycol | 3.0 |
| 9. Sodium citrate | 0.2 |
| 10. Preservative | appropriate amount |
| 11. Fragrance | appropriate amount |

(continued)

| (Components) | (parts by mass) |
|---|---|
| 12. Purified water | 22.8 |

(note 28: KF-6017, manufactured by Shin-Etsu Chemical Co., Ltd.)
(note 29: SPD-T5, manufactured by Shin-Etsu Chemical Co., Ltd.)
(note 30: SPD-Z5, manufactured by Shin-Etsu Chemical Co., Ltd.)

(Preparation method)

a: Components 1 to 5 were uniformly mixed by agitation.
b: Components 8 to 10 and component 12 were mixed.
c: "b" was added into "a", and then they were mixed by agitation for emulsification.
d: Components 6, 7, and 11 were added into "c", and then they were mixed by agitation.

(Result)

The sun-cut milky lotion thus obtained was excellent in cosmetic durability with a light and wide spreading property and without greasiness and stickiness.

As mentioned above, the cosmetic containing the paste organopolysiloxane elastomer composition of the present invention showed an excellent adhesion property and such feelings as a sleek and a refreshing feeling with a wide and a light spreading property and without such feelings as a greasy and a sticky feeling.

It must be noted here that the present invention is not limited to the embodiments shown above. The embodiments shown above are mere examples so that any embodiments composed of substantially the same technical concept as disclosed in the claims of the present invention and expressing a similar effect thereto are included in the technical scope of the present invention.

## Claims

**1.** A paste organopolysiloxane elastomer composition comprising at least:

(I) an organopolysiloxane elastomer microparticle obtained by an addition polymerization of an organopolysiloxane mixture containing:

(i) an organohydrogen polysiloxane having at least two silicon-bonded hydrogen atoms in its molecular structure and shown by the following general formula (1);

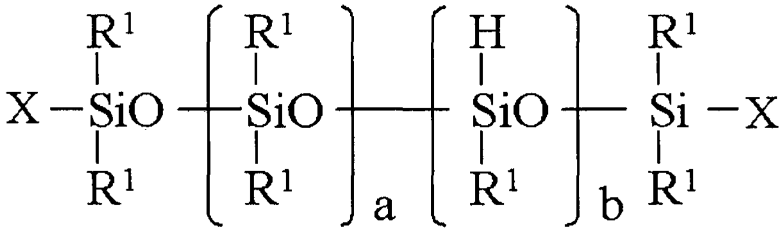

(1)

wherein each $R^1$ may be the same or different and represents a monovalent hydrocarbon group having 1 to 8 carbon atoms and not containing an aliphatic unsaturated group, wherein "a" represents an integer of one or more, and "b" represents an integer of 0 or more, with a+b being an integer of 80 or more, wherein each X may be the same or different, and represents a hydrogen atom or $R^1$, and

(ii) an organopolysiloxane having at least two aliphatic unsaturated groups in its molecular structure and shown by the following general formula (2);

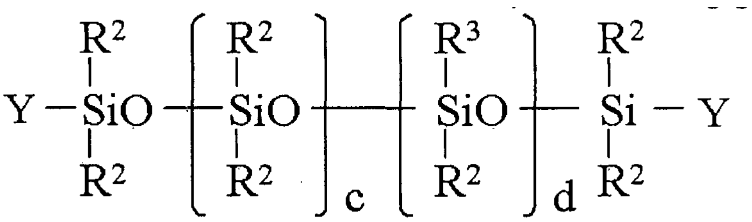 (2)

wherein each $R^2$ may be the same or different and represents a monovalent hydrocarbon group having 1 to 8 carbon atoms and not containing an aliphatic unsaturated group, and each $R^3$ may be the same or different and represents a monovalent hydrocarbon group having 2 to 8 carbon atoms and containing an aliphatic unsaturated group at its terminal, wherein "c" represents an integer of one or more, and "d" represents an integer of 0 or more, with c+d being an integer of 80 or more, wherein each Y may be the same or different, and represents $R^2$ or $R^3$; and

(II) an oil that is a liquid at 25°C; wherein
the organopolysiloxane elastomer microparticle of the component (I) is a spherical or an amorphous microparticle having a volume-average particle diameter of 2 to 50 μm and can absorb 200 or more parts by mass of a methyl polysiloxane having a dynamic viscosity of 10 $mm^2$/second or less at 25°C relative to 100 parts by mass of the organopolysiloxane elastomer microparticle and the ratio of the frictional resistance force of the oil which is used for the organopolysiloxane elastomer composition to the frictional resistance force of the organopolysiloxane elastomer composition is 0.80 or more, and wherein the volume-average particle diameter is measured by a Coulter-counter method and the frictional resistance force is measured by a frictional resistance force-measuring apparatus which is capable of, at the time of frictioning an evaluation sample on a sample holder, measuring a frictional force of horizontal direction relative to the holder and a load of vertical direction relative to the holder at the same time.

**2.** The organopolysiloxane elastomer composition according to claim 1, wherein the liquid oil of the component (II) is at least one kind selected from an silicone oil, a hydrocarbon oil, an ester oil, a natural animal and vegetable oil, and a semi-synthetic oil.

**3.** The organopolysiloxane elastomer composition according to claim 1 or 2, wherein $R^3$ is a vinyl group.

**4.** A cosmetic, wherein the organopolysiloxane elastomer composition according to any one of claims 1 to 3 is contained therein.

**Patentansprüche**

**1.** Organopolysiloxanelastomer-Pasten-Zusammensetzung, umfassend mindestens:

(I) ein Organopolysiloxanelastomer-Mikropartikel, das durch Additions-Polymerisation einer Organopolysiloxan-Mischung erhalten wurde, die enthält:

(i) ein Organowasserstoff-Polysiloxan mit mindestens zwei Siliziumverknüpften Wasserstoffatomen in seiner molekularen Struktur und das durch die folgende allgemeine Formel (1) gezeigt ist;

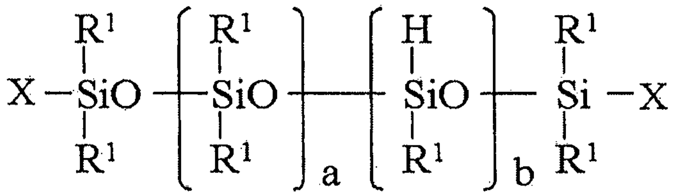 (1)

wobei jededs $R^1$ gleich oder verschieden sein kann und eine monovalente Kohlenwasserstoffgruppe mit 1 bis 8 Kohlenstoffatomen, die keine aliphatische ungesättigte Gruppe enthält, darstellt, wobei "a" eine Ganzzahl von eins oder mehr darstellt, und "b" eine Ganzzahl von 0 oder mehr darstellt, wobei a+b eine

Ganzzahl von 80 oder mehr sind, wobei jedes X gleich oder verschieden sein kann, und ein Wasserstoffatom oder $R^1$ darstellt, und

(ii) ein Organopolysiloxan mit mindestens zwei aliphatischen ungesättigten Gruppen in seiner molekularen Struktur und das durch die folgende allgemeine Formel (2) gezeigt ist;

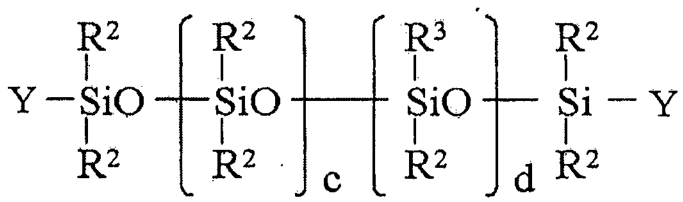

(2)

wobei jedes $R^2$ gleich oder verschieden sein kann und eine monovalente Kohlenwasserstoffgruppe mit 1 bis 8 Kohlenstoffatomen, die keine aliphatische ungesättigte Gruppe enthält, darstellt, und jedes $R^3$ gleich oder verschieden sein kann und eine monovalente Kohlenwasserstoffgruppe mit 2 bis 8 Kohlenstoffatomen, die eine aliphatische ungesättigte Gruppe an seinem Ende enthält, darstellt, wobei "c" eine Ganzzahl von eins oder mehr darstellt, und "d" eine Ganzzahl von 0 oder mehr darstellt, wobei c+d eine Ganzzahl von 80 oder mehr sind, wobei jedes Y gleich oder verschieden sein kann, und $R^2$ oder $R^3$ darstellt; und

(II) ein Öl, das eine Flüssigkeit bei 25°C ist; wobei
das Organopolysiloxanelastomer-Mikropartikel der Komponente (I) ein sphärisches oder ein amorphes Mikropartikel mit einem volumengemittelten Partikeldurchmesser von 2 bis 50 $\mu$m ist und 200 oder mehr Massenteile eines Methylpolysiloxans mit einer dynamischen Viskosität von 10 $mm^2$/Sekunde oder weniger bei 25°C, bezogen auf 100 Massenteile des Organopolysiloxanelastomer-Mikropartikels, absorbiert und das Verhältnis der Reibungswiderstandskraft des Öls, das für die Organopolysiloxanelastomer-Zusammensetzung verwendet wird, zu der Reibungswiderstandskraft der Organopolysiloxanelastomer-Zusammensetzung 0,80 oder mehr beträgt, und wobei der volumengemittelte Partikeldurchmesser durch ein Coulter-Counter-Verfahren gemessen wird und die Reibungswiderstandskraft durch ein Reibungswiderstandskraft-Messgerät gemessen wird, das fähig ist, während der Reibung einer zu beurteilenden Probe auf einem Probenhalter, eine Reibungskraft in horizontaler Richtung, bezogen auf den Halter, und eine Ladung in vertikaler Richtung, bezogen auf den Halter, zur gleichen Zeit zu messen.

**2.** Organopolysiloxanelastomer-Zusammensetzung nach Anspruch 1, wobei das flüssige Öl der Komponente (II) mindestens eines ist, das aus einem Siliziumöl, einem Kohlenwasserstofföl, einem Esteröl, einem natürlichen Tier- und Pflanzenöl, und einem semi-synthetischen Öl, ausgewählt ist.

**3.** Organopolysiloxanelastomer-Zusammensetzung nach Anspruch 1 oder 2, wobei $R^3$ eine Vinylgruppe ist.

**4.** Kosmetikartikel, wobei die Organopolysiloxanelastomer-Zusammensetzung gemäß einem beliebigen der Ansprüche 1 bis 3 darin enthalten ist.

## Revendications

**1.** Composition de pâte d'élastomère d'organopolysiloxane comprenant au moins :

(I) une microparticule d'élastomère d'organopolysiloxane obtenue par une polymérisation par addition d'un mélange d'organopolysiloxane contenant :

(i) un organohydrogénopolysiloxane ayant au moins deux atomes d'hydrogène liés au silicium dans sa structure moléculaire et représenté par la formule générale (1) suivante ;

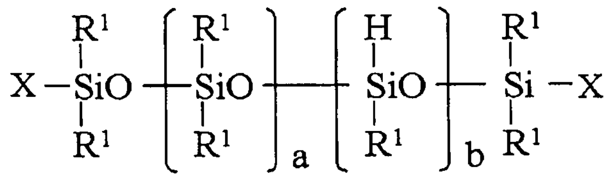

(1)

dans laquelle chaque $R^1$ peut être identique ou différent et représente un groupe hydrocarboné monovalent ayant 1 à 8 atomes de carbone et ne contenant pas de groupe aliphatique insaturé, dans laquelle « a » représente un nombre entier de un ou plus, et « b » représente un nombre entier de 0 ou plus, avec a + b étant un nombre entier de 80 ou plus, dans laquelle chaque X peut être identique ou différent, et représente un atome d'hydrogène ou $R^1$, et

(ii) un organopolysiloxane ayant au moins deux groupes aliphatiques insaturés dans sa structure moléculaire et représenté par la formule générale (2) suivante ;

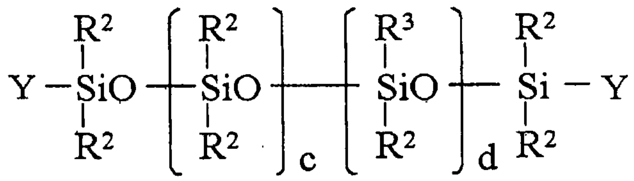

(2)

dans laquelle chaque $R^2$ peut être identique ou différent et représente un groupe hydrocarboné monovalent ayant 1 à 8 atomes de carbone et ne contenant pas de groupe aliphatique insaturé, et chaque $R^3$ peut être identique ou différent et représente un groupe hydrocarboné monovalent ayant 2 à 8 atomes de carbone, et contenant un groupe aliphatique insaturé à son extrémité, dans laquelle « c » représente un nombre entier de un ou plus, et « d » représente un nombre entier de 0 ou plus, avec c + d étant un nombre entier de 80 ou plus, dans laquelle chaque Y peut être identique ou différent, et représente $R^2$ ou $R^3$ ; et

(II) une huile qui est liquide à 25 °C ;
dans laquelle la microparticule d'élastomère d'organopolysiloxane du constituant (I) est une microparticule sphérique ou amorphe ayant un diamètre moyen de particule en volume de 2 à 50 $\mu$m et peut absorber 200 parties en masse ou plus d'un méthyle polysiloxane ayant une viscosité dynamique de 10 $mm^2$/seconde ou moins à 25 °C par rapport à 100 parties en masse de la microparticule d'élastomère d'organopolysiloxane et le rapport de la force de résistance au frottement de l'huile qui est utilisée pour la composition d'élastomère d'organopolysiloxane à la force de résistance au frottement de la composition d'élastomère d'organopolysiloxane est de 0,80 ou de plus, et dans laquelle le diamètre moyen de particule en volume est mesuré par une méthode par compteur Coulter et la force de résistance au frottement est mesurée par un appareil de mesure de force de résistance au frottement qui est apte, au moment du frottement d'un échantillon d'évaluation sur un support d'échantillon, de mesurer une force de frottement de la direction horizontale par rapport au support et une charge de la direction verticale par rapport au support en même temps.

2. Composition d'élastomère d'organopolysiloxane selon la revendication 1, dans laquelle l'huile liquide du constituant (II) est au moins un type choisi parmi une huile de silicone, une huile hydrocarbonée, une huile d'ester, une huile naturelle animale et végétale, et une huile semi-synthétique.

3. Composition d'élastomère d'organopolysiloxane selon la revendication 1 ou 2, dans laquelle $R^3$ est un groupe vinyle.

4. Produit cosmétique, dans lequel la composition d'élastomère d'organopolysiloxane selon l'une quelconque des revendications 1 à 3 est contenue dans celui-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2582275 B
- US 5654362 A
- JP 2005537364 W
- JP 3488573 B
- JP 4025454 B
- JP 2832143 B
- JP 2613124 B
- JP 3850202 B
- JP 3724988 B
- JP 3976226 B
- JP H0655897 B
- JP 2631772 B
- JP 3969728 B
- JP 4001342 B
- JP 4490817 B